# EUROPEAN PATENT APPLICATION

(11) **EP 2 458 517 A1**
(43) Date of publication of application: **30.05.2012**
(21) Application number: 11008209.6
(22) Date of filing: 09.12.2005
(51) Int. Cl.: G06F 19/00

(54) **Early detection of sepsis**

(30) Priority: 09.12.2004 GB 0426982
(62) Divisional of application: 05825137.2
(71) Applicant: The Secretary of State for Defence, Salisbury Wiltshire SP4 0JQ (GB)
(72) Inventor: JACKSON, Matthew, Christopher, Salisbury, Wiltshire SP4 0JQ (GB); LUKASZEWSKI, Roman, Antoni, Salisbury, Wiltshire SP4 0JQ (GB); YATES, Amanda, Marie, Salisbury, Wiltshire SP4 0JQ (GB); PEARCE, Martin, Julian, Salisbury, Wiltshire SP4 0JQ (GB); BROOKS, Timothy, John, Gilbert, Salisbury, Wiltshire SP4 0JQ (GB)
(74) Representative: Farnsworth, Alastair Graham

(57) **Abstract**

The invention describes a system and method for detecting early signs of infection and, in particular, for identifying individuals most likely to develop sepsis, Measurement of expression levels of particular combinations of cytokines and/or cellular activation markers, optionally combined with the use of predictive algorithms, allows a high degree of accuracy of prediction. The method is applicable in both civilian and military contexts.

## Description

### Background

Despite greatly improved diagnosis, treatment and support, serious infection and sepsis remain significant causes of death and often result in chronic ill-health or disability in those who survive acute episodes. Although sudden, overwhelming infection is comparatively rare amongst otherwise healthy adults, it constitutes an increased risk in immunocompromised individuals, seriously ill patients in intensive care, bums patients and young children. In a proportion of cases, an apparently treatable infection leads to the development of sepsis; a dysregulated, inappropriate response to infection characterised by progressive circulatory collapse leading to renal and respiratory failure, abnormalities in coagulation, profound and unresponsive hypotension and, in about 30% of cases death. The incidence of sepsis in the population of North America is about 0.3% of the population annually (about 750,000 cases) with mortality rising to 40% in the elderly and to 50% in cases of the most severe form, septic shock (Angus et al, 2001, Crit Care Med 29: 1303-1310).

Following infection with infectious micro-organisms, the body reacts with a classical inflammatory response and activation of, first, the innate, non-specific immune response, followed by a specific, acquired immune response. In the case of bacterial infections, bacteraemia leads to the rapid (within 30-90 minutes) onset of pyrexia and release of inflammatory cytokines such as interleukin-1 (IL-1) and tumour necrosis factor-α (TNF-α) triggered by the detection of bacterial toxins, long before the development of a specific, antigen-driven immune response.

In Gram-negative bacteraemia due to infections such as typhoid, plague, tularaemia and brucellosis, or peritonitis from Gram-negative gut organisms such as *Escherichia coli*, *Klebsiella*, *Proteus* or *Pseudomonas* this is largely a response to lipopolysaccharide (LPS) and other components derived from bacterial cell walls. Circulating LPS and, in particular, its constituent lipid A, provokes a wide range of systemic reactions. It is probably contact with Kupffer cells in the liver that first leads to IL-1 release and the onset of pyrexia. Activation of circulating monocytes and macrophages leads to release of cytokines such as IL-6, IL-12, IL-15, IL-18, TNF-α, macrophage migration inhibitory factor (MIF), and cytokine-like molecules such as high mobility group B1 (HMGB1), which, in turn activate neutrophils, lymphocytes and vascular endothelium, up-regulate cell adhesion molecules, and induce prostaglandins, nitric oxide synthase and acute-phase proteins. Release of platelet activating factor (PAF), prostaglandins, leukotrienes and thromboxane activates vascular endothelium, regulates vascular tone and activates the extrinsic coagulation cascade. Dysregulation of these responses results in the complications of sepsis and septic shock in terms of peripheral vasodilation leading to hypotension, and abnormal clotting and fibrinolysis producing thrombosis and intravascular coagulation (Cohen, 2002, Nature 420: 885-891).

LPS primarily acts on cells by binding to a serum LPS-binding protein (LBP) and CD14 expressed on monocytes and macrophages. On binding a complex of LPS and LBP, CD14 acts with a coreceptor, Toll-like receptor 4 (TLR-4) and a further component, MD-2, to form a signalling complex and initiate activation of macrophages and release of cytokines (Pålsson-McDermott & O'Neill, 2004, Immunology 113: 153-162). The Toll-like receptor family is a group of cell surface receptors involved in a range of bacterial and fungal ligands that act as triggers for innate immune system, including Gram-positive cell wall structures, flagellin, and CpG repeats characteristic of bacterial DNA.

In the case of infection with Gram-positive pathogens, septic shock is associated with the production of exotoxins. For instance, toxic shock syndrome, a particularly acute form of septic shock that often affects otherwise healthy individuals is due to infection with particular strain of *Staphylococcus aureus*, which produces an exotoxin known as toxic shock syndrome toxin-1 (TSST-1). A similar syndrome is caused by invasive infection with certain group A *Streptococcus* pyogenes strains, and is often associated with streptococcal pyogenic enterotoxin A (SPE-A). Some Gram-positive exotoxins (including TSST-1)are thought to exert their effects predominantly as a result of their superantigen properties. Superantigens are able to non-specifically stimulate T lymphocytes by cross-linking MHC Class II molecules on antigen presenting cells to certain classes of T cell receptors. Usually, T cell receptor (TCR) -Major Histocompatibility Complex (MHC) interactions are highly specific, with only T cells carrying TCRs that specifically recognise short antigen-derived peptides presented by the MHC able to bind and be activated, ensuring an antigen-specific T cell response. Superantigens bypass this mechanism resulting in massive and inappropriate activation of T cells. However, SPE-A is not an efficient superantigen and some further mechanism must be implicated.
It should be noted that clinical sepsis may also result from infection with some viruses (for example Venezuelan Equine Encephalitis Virus, VEEV) and fungi, and that other mechanisms are likely to be involved in such cases.

The ability to detect potentially serious infections as early as possible and, especially, to predict the onset of sepsis in susceptible individuals is clearly advantageous. A considerable effort has been expended over many years in attempts to establish clear criteria defining clinical entities such as shock, sepsis, septic shock, toxic shock and systemic inflammatory response syndrome (SIRS). Similarly, many attempts have been made to design robust predictive models based on measuring a range of clinical, chemical, biochemical, immunological and cytometic parameters and a number of scoring systems, of varying prognostic success and sophistication, proposed.

According to the 1991 Consensus Conference of the American College of Chest Physicians (ACCP) and Society of Critical Care Medicine (SCCM) "SIRS" is considered to be present when patients have more than one of the following: a body temperature of greater than 38°C or less than 36°C, a heart rate of greater than 90/min, hyperventilation involving a respiratory rate higher than 20/min or PaCO₂ lower than 32mm Hg, a white blood cell count of greater than 12000 cells /µl or less than 4000 cells /µl (Bone et al, 1992, Crit Care Med 20: 864-874).

"Sepsis" has been defined as SIRS caused by infection. It is accepted that SIRS can occur in the absence of infection in, for example, bums, pancreatitis and other disease states. "Infection" was defined as a pathological process caused by invasion of a normally sterile tissue, fluid or body cavity by pathogenic or potentially pathogenic micro-organisms.

"Severe sepsis" was defined as sepsis complicated by organ dysfunction, itself defined by Marshall et al (1995, Crit Care Med 23: 1638-1652) or the Sequential Organ Failure Assessment (SOFA) score (Ferreira et al, 2002, JAMA 286: 1754-1758).

"Septic shock" refers (in adults) to sepsis plus a state of acute circulatory failure characterised by a persistent arterial hypotension unexplained by other causes.

In order to evaluate the seriousness of sepsis in intensive care patients and to allow rational treatment planning, a large number of clinical severity models have been developed for sepsis, or adapted from more general models. The first generally accepted system was the Acute Physiology and Chronic Health Evaluation score (APACHE, and its refinements APACHE II and III) (Knaus et al, 1985, Crit Care Med 13: 818-829; Knaus et al, 1991, Chest 100: 1619-1636), with the Mortality Prediction Model (MPM) (Lemeshow et al, 1993, JAMA 270: 2957-2963) and the Simplified Acute Physiology (SAPS) score (Le Gall et al, 1984, Crit Care Med 12: 975-977) also being widely used general predictive models. For more severe conditions, including sepsis, more specialised models such as the Multiple Organ Dysfunction Score (MODS) (Marshall et al, 1995, Crit Care Med 23: 1638-1652), the Sequential Organ Failure Assessment (SOFA) score (Ferreira et al, 2002, JAMA 286: 1754-1758) and the Logistical Organ Dysfunction Score (LODS) (Le Gall et al, 1996, JAMA 276: 802-810) were developed. More recently, a specific model, PIRO (Levy et al, 2003, Intensive Care Med 29: 530-538), has been proposed. All of these models use a combination of a wide range of general and specific clinical measures to attempt to derive a useful score reflecting the seriousness of the patient's condition and likely outcome.

In addition to the standard predictive models described above, the correlation of sepsis and a number of specific serum markers has been extensively studied with a view to developing specific diagnostic and prognostic tests, amongst which are the following.

C-reactive protein (CRP) is a liver-derived serum acute phase protein that is well-known as non-specific marker of inflammation. More recently (Toh et al, 2003, Intensive Care Med 29: 55-61) a calcium dependent complex of CRP and very low density lipoprotein (VLDL), known as lipoprotein complexed C-reactive protein (LCCRP), has been shown to be involved in affecting the coagulation mechanism during sepsis. In particular, a common test known as the activated partial thromboplastin time develops a particular profile in cases of sepsis, and this has been proposed as the basis for a rapid diagnostic test.

TNF-α and IL-1 are archetypal acute inflammatory cytokines long known to be elevated in sepsis (Damas et al, 1989, Critical Care Med 17: 975--978) and have reported to be useful predictors of organ failure in adult respiratory distress syndrome, a serious complication of sepsis (Meduni et al, 1995, Chest 107: 1062-1073)

Activated complement product C3 (C3a) and IL-6 have been proposed as useful indicators of host response to microbial invasion, and superior to pyrexia and white blood cell counts (Groeneveld et al, 2001, Clin Diagn Lab Immunol 8: 1189-1195). Secretary phospholipase A₂ was found to be a less reliable marker in the same study.

Procalcitonin is the propeptide precursor of calcitonin, serum concentrations of which are known to rise in response to LPS and correlate with IL-6 and TNF-α levels. Its use as a predictor of sepsis has been evaluated (Al-Nawas et al, 1996, Eur J Med Res 1: 331-333). Using a threshold of 0.1 ng /ml, it correctly identified 39% of sepsis patients. However, other reports suggest that it is less reliable than the use of serial CRP measurements (Neely et al, 2004, J Burn Care Rehab 25: 76-80), although superior to IL-6 or IL-8 (Harbarth et al, Am J Resp Crit Care Med 164: 396-402).

Changes in neutrophil surface expression of leukocyte activation markers (such as CD11b, CD31, CD35, L-selectin, CD16) have been used as a marker of SIRS and have been found to correlate with IL-6 and subsequent development of organ failure (Rosenbloom et al, 1995, JAMA 274: 58-65). Similarly, expression of platelet surface antigens such as CD63, CD62P, CD36 and CD31 have been examined, but no reliable predictive model constructed.

Finally, it has been shown that downregulation of monocyte HLA-DR expression is a predictor of a poor outcome in sepsis and may be an indication of monocyte deactivation, impairing TNF-α production. Treatment with IFN-γ has been shown to be beneficial in such cases (Docke et al, 1997, Nature Med 3: 678-681).

However, although many of these markers correlate with sepsis and some give an indication of the seriousness of the condition, no single marker or combination markers has yet been shown to be a reliable diagnostic test, much less a predictor of the development of sepsis. The 2001 International Sepsis Definition Conference concluded that "the use of biomarkers for diagnosing sepsis is premature" (Levy et al, 2003, Intensive Care Med 29: 530-538).

Extracting reliable diagnostic patterns and robust prognostic indications from changes overtime in complex sets of variables including traditional clinical observations, clinical chemistry, biochemical, immunological and cytometric data requires sophisticated methods of analysis. The use of expert systems and artificial intelligence, including neural networks, for medical diagnostic applications has been being developed for some time (Place et al, 1995, Clinical Biochemistry 28: 373-389; Lisboa, 2002, Neural Networks 15: 11-39). Specific systems have been developed in attempts to predict survival of sepsis patients (Flanagan et al, 1996, Clinical Performance & Quality Health Care 4: 96-103) by use of multiple logistic regression and neural network models using APACHE scores and the 1991 ACCP/SCCM SIRS criteria described above (Bone et al, 1992, Crit Care Med 20: 864-874). Such studies suggest that, although both approaches can give good predictive results, neural network systems are less sensitive to preselected threshold values (results of a number of studies reviewed by Rosenberg, 2002, Curr Opin Crit Care 8:321-330). Brause et al (2004, Journal für Anästhesie und Intensivbehandlung 11: 40-43) provides an example of a neural network model being used for sepsis prediction. This model (MEDAN) analysed a range of standard clinical measure and compared its results with those obtained by using the APACHE II, SOFA, SAPS II, and MODS models. The study concluded that, of the markers available, the most informative were systolic and diastolic blood pressure, and platelet count.

Neural networks are non-linear functions that are capable of identifying patterns in complex data systems. This is achieved by using a number of mathematical functions that make it possible for the network to identify structure within a noisy data set. This is because data from a system may produce patterns based upon the relationships between the variables within the data. If a neural network sees sufficient examples of such data points during a period known as "training", it is capable of "learning" this structure and then identifying these patterns in future data points or test data. In this way, neural networks are able to predict or classify future examples by modelling the patterns present within the data it has seen. The performance of the network is then assessed by its ability to correctly predict or classify test data, with high accuracy scores, indicating the network has successfully identified true patterns within the data. The parallel processing ability of neural networks is dependent on the architecture of its processing elements, which are arranged to interact according to the model of biological neurones. One or more inputs are regulated by the connection weights to change the stimulation level within the processing element The output of the processing element is related to its activation level and this output may be non-linear or discontinuous. Training of a neural network therefore comprises an adjustment of interconnected weights depending on the transfer function of the elements, the details of the interconnected structure and the rules of learning that the system follows (Place et al, 1995, Clinical Biochemistry 28: 373-389). Such systems have been applied to a number of clinical situations, including health outcomes models of trauma patients (Marble & Healy (1999) Art Intell Med 15: 299-307).

Warner et al (1996, Ann Clin Lab Sci 26: 471-479) describe a multiparametric model for predicting the outcome of sepsis, using measures of 'septic shock factor' (which appears to be simply whether the patients have signs of septic shock on admission), IL-6, soluble II-6 receptor (as measured by enzyme-linked immunosorbent assay) and the APACHE II score as components of a four-input algorithm in a multi-layer, feed-forward neural network model. However, this system is not predictive for individuals who do not yet have clinical signs and, arguably, by the time serum levels of cytokines such as IL-6 are raised, the diagnosis, if not the outcome, is clinically obvious.

Dybowski et al (1996, Lancet 347: 1146-1150) use Classification and Regression Trees (CART) to select inputs from 157 possible sepsis prediction criteria and then use a neural network running a genetic algorithm to select the best combination of predictive markers. These include many routine clinical values and proxy indicators rather than serum or cell surface biomarkers. However, the problem being addressed is the prognosis of patients who already have a clear diagnosis of sepsis and are already critically ill.

A further refinement of the genetic algorithm approach involves the use of Artificial Immune Systems, of which one version is the Artificial Immune Recognition System (AIRS) (Timmis et al, An overview of Artificial Immune Systems. In: Paton, Bolouri, Holcombe, Parish and Tateson (eds.) "Computation in Cells and Tissues: Perspectives and Tools for Thought", Natural Computation Series, pp51-86, Springer, 2004; Timmis (L.N. De Castro and J, Timmis. Artifical Immune Systems: A New Computational Intelligence Approach. Springer-Verlag, 2002).which are adaptive systems inspired by the clonal selection and affinity maturation processes of biological immune systems as applied to artificial intelligence.

Immunologically speaking, AIRS is inspired by the clonal selection theory of the immune system (F. Burnett. The Clonal SelectionTheory of Acquired Immunity. Cambridge University Press, 1959). The clonal selection theory attempts to explain that how, through a process of matching, cloning, mutation and selection, anti-bodies are created that are capable of identifying infectious agents. AIRS capitalises on this process, and through a process of matching, cloning and mutation, evolves a set of memory detectors that are capable of being used as classifiers for unseen data items. Unlike other immune inspired approaches, such as negative selection, AIRS is specifically designed for use in classification, more specifically one-shot supervised learning.

US patent application 2002/0052557 describes a method of predicting the onset of a number of catastrophic illnesses based on the variability of the heart-rate of the patient. Again, a neural network is among the possible methods of modelling and analysing the data.

International patent application WO 00/52472 describes a rapid assay method for use in small children based on the serum or neutrophil surface levels of CD11b or 'CD11b complex' (Mac-1, CR3). The method uses only a single marker, and one which is, arguably, a well-known marker of neutrophil activation in response to inflammation.
The alternative approach to analysing such complex data sets where the data are often qualitative and discrete, rather than quantitative and continuous, is to use sophisticated statistical analysis techniques such as logistic regression. Where logistic regression using qualitative binary dependent variables is insufficiently discriminating in terms of selecting significant variables, multivariate techniques may be used. The outputs from both multiple logistic regression models and neural networks are continuously variable quantities but the likelihoods calculated by neural network models usually fall at one extreme or the other, with few values in the middle range. In a clinical situation this is often helpful and can give clearer decisions (Flanagan et al, 1996, Clinical Performance & Quality Health Care 4: 96-103).

The ability to detect the earliest signs of infection and / or sepsis has clear benefits in terms of allowing treatment as soon as possible. Indications of the severity of the condition and likely outcome if untreated inform decisions about treatment options. This is relevant both in vulnerable hospital populations, such as those in intensive care, or who are burned or immunocompromised, and in other groups in which there is an increased risk of serious infection and subsequent sepsis. The use or suspected use of biological weapons in both battlefield and civilian settings is an example where a rapid and reliable means of testing for the earliest signs of infection in individuals exposed would be advantageous.

### Statement of Invention

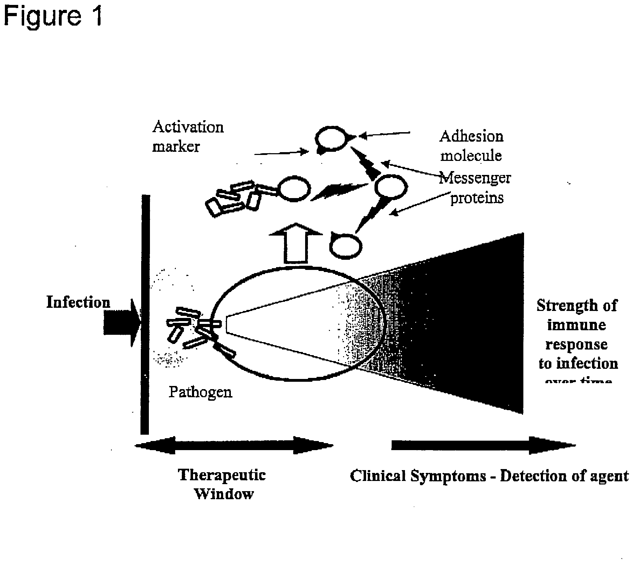

Following infection, cells of the immune system recognise and respond to a pathogen by becoming activated. This results in the production of different messenger proteins (e.g. cytokines and chemokines) and expression of activation markers and adhesion molecules on the cell surface (Figure 1). The production of these facilitates communication between cells and results in a coordinated immune response against a particular agent. Since this inflammatory immune response is relatively constant in response to infection, and occurs in the very earliest stages of the disease process, monitoring changes in the expression of such markers predict the early stages of sepsis development. It is an object of the invention to provide a means of detecting serious infections at an early stage, preferably, during the therapeutic window of intervention, prior to the onset of clinical symptoms and disease (Figure 1).

In a first aspect, the invention describes a system and methods of detecting early signs of infection, SIRS or sepsis several days before clinical signs become apparent. It also provides methods capable of predicting the timing of the clinical course of the condition. The system comprises analysing the results of one or more sets of tests based on biological samples, preferably blood samples. Optionally, other routine clinical measurements may be included for analysis.

The first set of test comprises determining the level of expression of a panel of chemokines and cytokines in blood leukocytes by amplifying cytokine mRNA by reverse transcription polymerase chain reaction (RT-PCR). This group comprises CD178 (FAS-L), MCP-1 (monocyte chemotactic protein-1), TNF-α, IL-1β, IL-6, IL-8, IL-10, INF-α and INF-γ. CD178 is encoded and expressed as a type-II membrane protein, but may be considered as a cytokine since it is cleaved by a metalloprotease to release a soluble homotrimer, soluble FasL or sFasL.

The second set of tests comprises determining cell surface expression of a group of surface markers comprising HLA-DR, CD54, CD11b, CD31, CD69, CD80, CD62L (L-selectin), CD25 and CD97. This may done by any standard method, preferably by labelling the cells with specific, labelled antibodies or fragments thereof and analysing by flow cytometry.

A third set of tests that may be included in the analysis comprise routine clinical data including temperature, heart rate, total and differential white blood cell count (monocytes, lymphocytes, granulocytes, neutrophils), platelet count, serum creatinine, urea, lactate, base excess, pO₂, HCO₃⁻ , and C-reactive protein.

The results of one or more sets of tests are analysed, preferably by means of a neural network program enabling a yes /no prediction of the patient from whom the sample was taken developing sepsis to be calculated.

In the case of a positive prediction, preferably a further analysis is then performed allowing an estimate to be made as to the time to onset of overt clinical signs and symptoms. Alternatively, both analyses may be performed by multivariate logistic regression.

Analysis of the test groups can be performed individually or simultaneously. Preferably clinical data are entered into the neural net as supplementary data to the PCR data. At the same time flow cytometry data can be processed by the neural network. Only one set of data is required for processing through the neural net although there are advantages in inputting one, two or all three data sets as these additional examples help "train" the neural net and improve confidence in the output from the program.

In a further aspect the neural network is used to process pre-recorded clinical data or a database of such data may be used to train the neural network and improve its predictive power.

The method may be used as part of routine monitoring for intensive care patients, where regular blood samples are taken for other purposes. Other hospital patients who may be predisposed to infections and/or sepsis may also be monitored. Such predisposing conditions include inherited or acquired immunodeficiencies (including HIV/AIDS) or immunosuppression (such as general surgery patients, transplant recipients or patients receiving steroid treatment), diabetes, lymphoma, leukaemia or other malignancy, penetrating or contaminated trauma, burns or peritonitis. In another aspect, the method of the invention may be used to screen individuals during an outbreak of infectious disease or alternatively individuals who have been, or who are suspected of having been, exposed to infectious pathogens, whether accidentally or deliberately as the result of bioterrorism or of use of a biological weapon during an armed conflict.

The invention therefore provides a method for screening a biological sample to detect early stages of infection, SIRS or sepsis comprising the steps of: detecting expression of a set of informative biomarkers by RT-PCR and/or detecting expression of a set of informative cell surface biomarkers by means of flow cytometry and/or monitoring a set of standard clinical measurements; analysing the results of detection by means of a neural network or multivariate statistical analysis; classifying said sample according to the likelihood and timing of the development of overt infection. Preferably, said biological sample is a blood sample. Further preferably, the results of the RT-PCR and/or the flow cytometry and/or standard clinical measurements are subjected to a first analysis yielding a prediction as to the probability of SIRS or sepsis developing. In one alternative embodiment, this is expressed as a probability. In an alternative embodiment it is expressed as a binary yes/no result.

Optionally, where the first analysis suggests that SIRS or sepsis are probable (as defined as exceeding a predetermined arbitrary threshold probability, or a 'yes' prediction, said results are subjected to a second analysis to determine the likely time to development of overt clinical signs, or to give an indication of probable severity of the clinical disease.

Preferably, the set of informative biomarkers, expression of which is detected by means of RT-PCR consists of at least 4, preferably at least 6, more preferably at least 7, most preferably all selected from the list consisting of CD178 (FasL), MCP-1 (alternatively known as CCL-2), TNFα, IL-1β, IL-6, IL-8, IL-10, INF-α and INF-γ.

Alternatively, the set of informative cell surface biomarkers the expression of which is detected by means of flow cytometry consists of at least one, preferably at least two and most preferably at least three selected from the list consisting of CD31, HLA-DR, CD54, CD11b, CD62L, CD 25, CD69, CD80 and CD97.

Where standard clinical measurement are analysed these are consist of at least one, preferably at least three, more preferably at least five selected from the list consisting of temperature, heart rate, total and differential white blood cell count (monocytes, lymphocytes, granulocytes, neutrophils), platelet count, serum creatinine, urea, lactate, base excess, pO₂, HCO₃⁻, and C-reactive protein

In one highly favoured embodiment, said analysis is by means of a neural network. Most preferably it is a multilayered perceptron neural network

Preferably such a neural network is capable of correctly predicting SIRS or sepsis in greater than 70% of cases (determined in trials where such development is not prevented by prophylactic treatment in a control group), more preferably in at least 80% of cases, even more preferably in at least 85% of cases and most preferably in at least 95% of cases. It is preferred that SIRS or sepsis is can be predicted at least one day before the onset of overt clinical signs, more preferably, at least two days, still more preferably at least three days and most preferably more than three days before SIRS or sepsis is diagnosed.

In another favoured embodiment analysis is by means of multivariate statistical analysis, preferably comprising principle component analysis and/or discriminant function analysis. It is more preferred that the multivariate statistical analysis comprises discriminate function analysis.

In a further aspect, the invention provides a system for screening a biological sample to detect early stages of infection, SIRS or sepsis comprising: a means of extracting and purifying RNA from cells obtained from said sample, a thermal cycler or other means to amplify selected RNA sequences by means of reverse transcription polymerase chain reaction (RT-PCR), a means of detecting and quantifying the results of said RT-PCR, a computer-based neural network trained so as to be able to analyse such results and a display means whereby the conclusion of the neural network analysis may be communicated to an operator.
Note: in this aspect the results of the RT-PCR may be analysed using discriminate function analysis, but the neural network is the preferred embodiment.

Alternatively, system comprises: a means of labelling specific cell surface markers on cells obtained from a biological sample and quantifying expression of said markers, a means of detecting and quantifying the results of said labelling and quantification, a computer-based neural network trained so as to be able to analyse such results and a display means whereby the conclusion of the neural network analysis may be communicated to an operator. Preferably, the labelling is by means of labelled antibodies or antibody fragments and the quantification is by means of fluorescence-activated cell sorting (FACS) or other form of flow cytometry.

Preferably, said system comprises means for both RT-PCR and FACS analysis and more preferably can also analyse other routine clinical test results, integrating all such results into its analysis.

In a further aspect the invention provides analysis according to any of embodiment of the method described above for the preparation of a diagnostic means for the diagnosis of SIRS, sepsis or infection, or the use of the system described above for the preparation of a diagnostic means for the diagnosis of infection.

Also provided is a method of early diagnosis of SIRS, infection or sepsis according to the method as described above.

### Detailed description of the invention

The invention will be described in further detail with reference to the following Figures and Examples
**Figure 1****:** Following infection, cells of the immune system recognise and respond to a pathogen by becoming activated. This results in the production of different messenger proteins (e.g. cytokines and chemokines) and expression of activation markers and adhesion molecules on the cell surface. The production of these facilitates communication between cells and results in a coordinated immune response against a particular agent. Since this inflammatory immune response is relatively constant in response to infection, and occurs in the very earliest stages of the disease process, monitoring changes in the expression of such markers can be used to predict the early stages of sepsis development. Ideally this is done during the therapeutic window of intervention, prior to the onset of clinical symptoms and disease.
**Figure 2****: A plot of the CD31 expression measured on granulocytes by flow cytometry.** From blood samples taken from patients three days before diagnosis of sepsis (n=6), and in ICU patients who did not go on to develop sepsis (n=24). Each symbol represents a measurement from one patient.
**Figure 3****: Design of neural network analysing clinical data according to Table 4, model 2.** WCC= white cell count, CRP= C-reactive protein.
**Figure 4****: Change in cytokine profile obtained following *in vitro* blood infection with *S. aureus*.** Data from blood taken from three volunteers as detailed in Example 8.
**Figure 5****: Results of neural network analysis of *S. aureus in vitro* sepsis model.**

### Example 1: Prediction of sepsis by neural network analysis of cytokine expression, cell surface markers and clinical measures.

### Study design and patients

The study into the onset of sepsis from the ICU department of Queen Alexandra hospital resulted in a cohort of ninety-one patients (Dstl/CR08631). Blood samples were collected daily from these patients throughout their stay in the ICU and in total, twenty-four patients were diagnosed as developing sepsis. Samples taken on the day clinical sepsis was diagnosed (Day 0), back through to six days prior to sepsis diagnosis (Day -6) were analysed by RT-PCR and flow cytometry for the expression of activation markers and cytokine mRNA respectively. In addition, standard hospital data and clinical observations were recorded. Samples from control patients were also processed in the same manner to provide data for traditional statistical analysis.

RT-PCR was performed according to commonly-used laboratory techniques. Briefly, in the case of a blood sample, whole blood was taken and cells then lysed in the presence of an RNA stabilising reagent. RNA was separated by affinity binding of beads, which were isolated by centrifugation (or magnetically, as appropriate), contaminating DNA removed by DNase digestion and the RNA subjected to RT-PCR.

Fluorescence activated cell sorting (FACS) flow cytometry is very well-known in the art and any standard technique may be used.

### Data analysis

The complexity of biological systems and intricate relationships between the markers used in this study caused standard linear techniques of data analysis to give inconclusive results. Consequently it was unclear whether any patterns existed in the data and a more powerful technique, capable of non-linear modelling, was sought to cope with the complexity of the data sets.

For analysis, data was collated from patients 1 to 4 days prior to the onset of sepsis and compared with an age/sex matched control group consisting of ICU patients who did not develop sepsis. Individual samples provided data measuring up to 56 different parameters and selective combinations of variables were fed into a multi-layered perceptron neural network (Proforma, Hanon Solutions, Glasgow, Scotland).

Each network was trained with a random 70% selection of balanced sepsis and control data using back propagation algorithms and then tested with the remaining 30% of the data. Five attempts were made at modelling the data within this network, each model differing in its ability to generalise to the data. The most successful model was the one most capable of correctly classifying previously unseen patients as being from either the sepsis or non-sepsis control group.

### Results

Table 2 shows an example of a successful model that classified or "scored" 29/35 (or 82.9%) test patients correctly.

**Table 2. Classification readout using cytokine mRNA variables (Days 1 to 4)**

| | Score | % of sample correctly predicted |
|---|---|---|
| Total patients | 29/35 | 82.9% |
| | | |
| Control patients | 16/20 | 80.0% |
| Sepsis patients | 13/15 | 86.7% |

To increase confidence in this model, this was carried out five times, each time using a different random selection of data for which to train and test the network. Once completed, the scores for the individual models were averaged to give an overall indication of the networks ability to classify patients into the correct sepsis or non-sepsis control group.

A series of 5 datasets gives a mean accuracy of prediction of approximately 80%, as shown by Table 3 below

**Table 3: neural network predicting sepsis using RT-PCR data only (Classification Performance Analysis of 5 projects)**

| | **Hits/Occurred** | **%** | **Hits/Predicted** | **%** | **Chance %** | **Improvement %** | **Ratio** |
|---|---|---|---|---|---|---|---|
| **MODEL 1** | | | | | | | |
| Condition | 23127 | 85.2 | N/A | N/A | 50.0 | 35.2 | 1.7:1 |
| Sepsis | 9/13 | 69.2 | 9/9 | 100.0 | 48.1 | 51.9 | 2.1:1 |
| Control | 14/14 | 100.0 | 14/18 | 77.8 | 51.9 | 25.9 | 1.5:1 |

| **MODEL 2** | | | | | | | |
|---|---|---|---|---|---|---|---|
| Condition | 29/35 | 82.9 | N/A | N/A | 50.0 | 32.9 | 1.7:1 |
| Sepsis | 13/15 | 86.7 | 13/17 | 76.5 | 42.9 | 33.6 | 1.8:1 |
| Control | 16/20 | 80.0 | 16/18 | 88.9 | 57.1 | 31.7 | 1.6:1 |

| **MODEL 3** | | | | | | | |
|---|---|---|---|---|---|---|---|
| Condition | 32/40 | 80.0 | N/A | N/A | 50.0 | 30.0 | 1.6:1 |
| Sepsis | 20/22 | 90.9 | 20/26 | 76.9 | 55.0 | 21.9 | 1.4:1 |
| Control | 12/18 | 66.7 | 12/14 | 85.7 | 45.0 | 40.7 | 1.9:1 |

| **MODEL 4** | | | | | | | |
|---|---|---|---|---|---|---|---|
| Condition | 21/26 | 80.8 | N/A | N/A | 50.0 | 30.8 | 1.6:1 |
| Sepsis | 9/12 | 75.0 | 9/11 | 81.8 | 46.2 | 35.7 | 1.8:1 |
| Control | 12/14 | 85.7 | 12/15 | 80.0 | 53.8 | 26.2 | 1.5:1 |

| **MODEL 5** | | | | | | | |
|---|---|---|---|---|---|---|---|
| Condition | 23/29 | 79.3 | N/A | N/A | 50.0 | 29.3 | 1.6:1 |
| Control | 12/15 | 80.0 | 12/15 | 80.0 | 51.7 | 28.3 | 1.5:1 |
| Sepsis | 11/14 | 78.6 | 11/14 | 78.6 | 48.3 | 30.3 | 1.6:1 |

128 / 157 = **81.5%**
79.3 + 85.2 + 80 + 82.9 + 80.8 = 408.2 / 5 = **81.64%**

Table 4 lists the averaged prediction accuracy values for a range of networks constructed using differing combinations of variables.

The most successful model was constructed using cytokine mRNA expression combined with CD31 % expression from the flow cytometry data (average 81.0% accuracy, Table 3, model 1) with clinical data also scoring highly (80.4%, Table 3, Model 2).

**Table 4. The results from the neural network analysis.**

| Model | Markers | Prediction Accuracy (%) |
|---|---|---|
| 1 | FasL, MCP-1, TNF-α, IL-1β, IL-6, IL-8, IL-10 | 81.6 |
| 2 | Creatinine, Monocytes, CRP, Lymphocytes, Temperature, Neutrophils, White Cell Count | 80.4 |
| 3 | FasL, MCP-1, IL-8, White cell count, Temperature, Creatinine | 79.0 |
| 4 | FasL, MCP-1, TNF-α, IL-1β, IL-6, IL-8 & IL-10, %CD31 & Creatinine | 78.7 |
| 5 | FasL, TNF-α, IL-1β, IL-6, IL-8 & IL-10 | 78.1 |
| 6 | FasL, MCP-1, TNF-α, IL-1β, IL-6, IL-8, IL-10, Creatinine, Monocytes, CRP, Lymphocytes, Temperature, Neutrophils, White Cell Count | 76.0 |
| 7 | MCP-1, TNF-α, IL-1β, IL-6, IL-8, IL-10 | 76.0 |
| 8 | Platelets, HCO3, PO2, Urea, Creatinine, heart rate | 70.7 |

To further test our predictive model, we trained the network on up to 100% of the cytokine data obtained from 1 to 4 days prior to the onset of clinical symptoms. We then selected test data comprising "Day 0" sepsis patients and those from Day -5 and Day -6. day 0, 5 and 6 and also selected 14 control patients from a separate volunteer study, 7 of which developed symptoms of an Upper Respiratory Tract Infection (URTI) within 9 days of sampling (Dstl/CR08631). The results are shown below in table 5.

**Table 5. Performance of cytokine mRNA model (Days -4 to -1) in prediction of other groups**

| Test set | Score | % of sample correctly predicted |
|---|---|---|
| Day 0 sepsis | 8/9 | 89% |
| Day -5 sepsis | 7/9 | 78% |
| Day -6 sepsis | 5/6 | 83% |

This table shows that our model, built from patterns expressed by sepsis patients up to 4 days before the onset of clinical sepsis, correctly identified, or "scored", 89% of day 0 sepsis patients, 78% of Day -5 sepsis patients and 83% of Day -6 sepsis patients. Overall, analysis using neural networks has led to the creation of a number of predictive models for sepsis. Models built using only cytokine data have proved consistently capable of successfully distinguishing between individuals who will develop sepsis from those that will not.

### Example 2: Lack of false positive results from non-sepsis volunteers using neural network model

Table 6 shows the results of testing a group of volunteers by cytokine RT-PCR, none of whom developed signs of SIRS or sepsis.

**Table 6**

| Name | Hits/Occurred | % | Hits/Predicted | % | Chance | Improvement | Ratio |
|---|---|---|---|---|---|---|---|
| Total | 13/13 | 100.0 | N/A | N/A | 50.0% | 50.0% | 2.0:1 |
| Control | 13/13 | 100.0 | 13/13 | 100.0 | 100.0% | 0.0% | 1.0:1 |
| Sepsis | 0/0 | N/A | 0/0 | 0.0 | 0.0% | 0.0% | N/A |

### Example 3: Neural network sepsis prediction of more than 90% accuracy using Clinical Data.

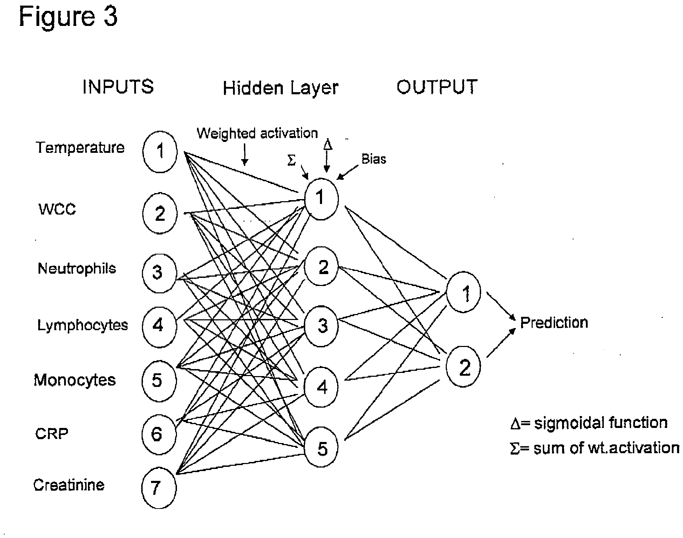

Neural network model tested using clinical data set defined in Table 4 model 2, using the parameters as described in Table 7 below and further illustrated in Figure 3:

**Table 7: Neural network parameters to analyse clinical data**

| Weights from input to hidden | | | | | | |
|---|---|---|---|---|---|---|
| Input | Unit 1 | | Unit 2 | Unit 3 | Unit 4 | Unit 5 |
| 1 | -3.04389 | | 0.783085 | -8.14579 | -5.31918 | -11.699 |
| 2 | -2.40492 | | -3.28341 | 6.81119 | -2.22889 | 10.3357 |
| 3 | 2 0.0835039 | | -2.25136 | 15.2575 | -3.38677 | 11.4842 |
| 4 | -16.2918 | | 3.45666 | -5.86258 | 8.93773 | -1.57967 |
| 5 | 12.7828 | | 45.2618 | 6.36791 | 7.45053 | 17.1156 |
| 6 | 34.3201 | | 7.31471 | -18.392 | 16.806 | 10.2057 |
| 7 | -3.31337 | | -1.41443 | -10.2639 | -3.68324 | 1.4483 |

| Weights from bias to hidden | | | | | | |
|---|---|---|---|---|---|---|
| 1 | | -0.407035 | -9.38879 | 4.06257 | -12.6877 | -10.7582 |
| Weights from hidden to output | | | | | | |
| 1 | | -8.79483 | 8.82026 | | | |
| 2 | | 8.79794 | -8.38064 | | | |
| 3 | | 3.60783 | -3.62177 | | | |
| 4 | | 3.00073 | -3.83778 | | | |
| 5 | | 4.85609 | -4.85559 | | | |

| Weights from bias to output | | | | | | |
|---|---|---|---|---|---|---|
| 1 | | -2.64015 | 2.65373 | | | |

**Table 8**

| Name | Hits/Occurred | % | Hits/Predicted | % | Chance | Improvement | Ratio |
|---|---|---|---|---|---|---|---|
| Condition | 14/15 | 93.3 | N/A | N/A | 50.0% | 43.3% | 1.9:1 |
| Sepsis | 8/8 | 100.0 | 8/9 | 88.9 | 53.3% | 35.6% | 1.7:1 |
| Control | 6/7 | 85.7 | 6/6 | 100.0 | 46.7% | 53.3% | 2.1:1 |

### Example 4: Use of Artificial Immune Recognition System

### Representation

The initial AIRS system (A. Watkins. *An Artificial Immune Recognition System*. Mississippi Sate University: MSc Thesis-, 2001) employed simple real-value shape space. Recently, other people have extended the representation to Hamming shape space (J. Hanamaker and L. Boggess. The effect of distance metrics on AIRS. In Proc. Of Congress on Evolutionary Computation (CEC). IEEE, 2004) and natural language (D. Goodman, L. Boggess and A. Watkins. "An investigation into the source of power for AIRS, an artificial immune classification system". In Proc. Int Joint Conference on Neural Networks, pp1678-1683. IEEE, 2003). AIRS maintains a set of Artificial Recognition Balls (ARBs) that contain a vector of the data being learnt, a stimulation level and a number of resources. During training, the stimulation level is calculated by assessing the affinity of the data vector in the ARB against a training item, the stronger the match, the greater the stimulation. This stimulation level is used to dictate how many clones the ARB will produce, and affects survival of the ARB.

### Affinity Measure

This is dependent on the representation employed. A number of affinity measures for use in AIRS have been proposed, including Hamming distance, Euclidean distance and so on. In this study, both Euclidean and Hamming distance metrics were used, with Euclidean giving the best results.

### Immune Algorithm

Essentially, AIRS evolves with two populations, a memory pool and an ARB pool **C.** It has a separate training and test phase, with the test phase being akin to a k-nearest neighbour classifier. During the training phase, a training data item is presented to **M.** This set can be seeded randomly, and experimental evidence would suggest that AIRS is insensitive to the initial starting point. The training item is matched against all memory cells in the set **M,** and a single cell is identified as the higher match *MCmatch.* This *MCmatch* is then cloned and mutated. Cloning is performed in proportion to stimulation (the higher the stimulation, the higher the clonal rate), and mutation is inversely proportional (the higher the stimulation, the lower the mutation rate). These clones are inserted into the ARB pool, **C.** The training item is then presented to the members of the ARB pool, where an iterative procedure is adopted which allows for the cloning and mutation of new candidate memory cells. Through a process of population control, where survival is dictated by the number of resources an ARB can claim, a new candidate memory cell is created. This mechanism is based on the resource allocation algorithm proposed in J. Timmis and M. Neal. A Resource Limited Artificial Immune System. Knowledge Based Systemsm 14(3/4): 121-130, 2001. This new candidate is compared against the *MCmatch*, with the training item. If the affinity between the candidate cell and *MVCmatch* is higher, then the memory cell is replaced with the candidate cell.

This process is performed for each training item, whereupon the memory set will contain a number of cells capable of being used for classification. Classification of an unseen data item is performed in a k-nearest neighbour fashion.

### Experimental Setup

An attempt was made to use an experimental procedure that was comparable to the application of neural networks to this data set. For all studies, the marks: asL, MCP-1, TNF-α, IL-β, IL-6, IL-8 and IL-10 were used. However, it was not possible to completely reproduce exactly the data set, due to incomplete information regarding the pre-processing of the data during the neural network study.

### Experiment One

In the first set of experiments, data collected from patients on days 1 through 4 prior to the onset of sepsis, along with data from a control set of patents as training data were used. Specifically, the combined data from days 1, 2, 3 and 4 for patients who showed signs of sepsis were used, and a random collection of control patients in order to train AIRS. In total, 59 training data items were used. To test AIRS, a random collection of patients from the control group and combined data from all days (excluding data that had been used in the training process) was used. In total, 34 test data points were used. The settings for AIRS are shown in table 9:

**Table 9: Parameter Settings for AIRS**

| Parameter | Setting |
|---|---|
| Epochs | |
| Clonal Rate | 10 |
| Mutation Rate | 0.8 |
| Initial size of population | 5 |
| Affinity Threshold | 0.2 |
| Stimulation Threshold | 0.8 |
| Number of resources | 200 |

### Experiment Two

For our second set of experiments, patients were classified who showed signs of sepsis using data for days 0, 5 and 6 and control patients. The AIRS system was trained using the same data as for Experiment One, whilst making use of the same parameters.

### Results

The results are not directly comparable with the results obtained from the neural network analysis due to the fact thatit was difficult to ascertain from the original report exactly how the data had been first combined over a period of days, and then divided into training and test sets. Therefore, the results obtained should be considered with this in mind.

### Experiment One

Ten independent runs of the AIRS algorithm were run, then the average and standard deviation calculated. It was found that AIRS was capable of achieving on average 73(2.96)% classification accuracy. This is approximately 10% lower than the neural network analysis, (using the same markers). However, care has to be taken with a direct comparison.

### Experiment Two

Again, ten independent runs of the AIRS algorithm were undertaken, and the average and standing deviation taken. This time, preceding days (0, 5 and 6) before the onset of sepsis were analysed, and the control group. Again, AIRS was trained on data taken from days 1 through 4 and the control group. These results are presented in Table 10.

**Table 10: Prediction in Other Groups (standard deviation in braces)**

| Test Set | Accuracy |
|---|---|
| Day 0 sepsis | 83(7.6)% |
| Day 5 sepsis | 93(6.1)% |
| Day 6 sepsis | 91(8.2)% |
| Control | 70(12.8)% |

As can be seen from Table 10, AIRS identifies a high percentage of sepsis cases (being able to outperform the neural network on day 5 and day 6, but again with the comparative caveat). The control group did not fair as well, being a lower than expected result, and significantly lower than the neural network approach. This may be due to the fact that AIRS has biased towards the sepsis patients due to the larger amount of data available for training with those, than for non-sepsis.

### Conclusions

AIRS appears capable of identifying potential cases of sepsis in advance, and comparable at a certain level to neural network approaches.

### Example 5: Use of CD31 expression to predict sepsis

### Study design and patients

See Example 1.

### Flow Cytometry

Blood was collected into sodium heparin containers (HM&S, Chessington, Surrey) and transported to the laboratory at room temperature. 100 µl aliquots of blood were mixed with immunofluorescent stains using the volumes recommended by the manufacturer (Beckman Coulter limited, High Wycombe, Buckinghamshire, and Becton Dickinson UK Limited, Cowley, Oxford). T helper cells were identified by co-staining for CD3 and CD4 and T cytotoxic cells were identified by staining for both CD3 and CD8. These cell populations were stained for HLA-DR, CD25, CD54 and CD69. B cells were identified by staining for CD19 and were interrogated with CD80, CD86, CD25 CD54. Natural killer cell were distinguished by staining with CD56 and interrogated with CD11b, CD25, CD54 and CD69. The monocyte population was selected by staining for CD14, these cells were probed with CD11b, CD54, CD80, CD86 and HLA-DR stains. Gating was used in order to identify the granulocyte population, which was stained for CD11b, CD69, CD31, CD54 and CD62L. The stains were incubated at room temperature for 20 minutes. 500 µl of Optilyse C (Beckman Coulter limited) was added to each tube and vortex mixed immediately. The samples were incubated at room temperature for 10 minutes to lyse the red blood cells and 500 µl of Isoton (Beckman Coulter limited) were then added in order to fix the stains. The tubes were vortex mixed immediately and incubated at room temperature for 10 minutes. The cells were then counted on a Beckman Coulter Epics XL System 2 Flow Cytometer.

StatisticsData was analysed using a Binary Logistic Regression model on the SPSS software package version 11.0. This analysis compared the control group means for immune modulator expression with the means obtained from the sepsis patients at seven time points: 6 days before diagnosis, 5, 4, 3, 2, and 1 day before diagnosis and 0, on the day of diagnosis of sepsis. Where data points were missing, averaged values for the group were substituted in order to maintain acceptable n values. Results from the model were only reported if the substituted data points did not involve markers that were highlighted by the model as possible predictors.

### Results

Analysis of the data found that there was weak evidence of a predictor effect = 0.114. Decreased expression of CD31 was indicated to be a possible predictor of sepsis three days before diagnosis p=0.037 (n=6). The results obtained for 6 days before diagnosis were inconclusive because of the small sample size for this date (n=4). There were no statistically significant predictors found for 5, 4, 2 or 1 day before diagnosis, or for the day of diagnosis.

### Conclusions

The flow cytometry data obtained from patients prior to the development of sepsis, and from patients who did not develop this disease were collated. Groups were constructed using results from patients in the days before diagnosis of sepsis, with a control group consisting of measurements taken from age matched patients who did not develop sepsis. Examination of bar graphs displaying the medians and 90^{th} and 10^{th} percentiles were difficult to interpret because of the spread of the data and hence statistical analysis was performed.

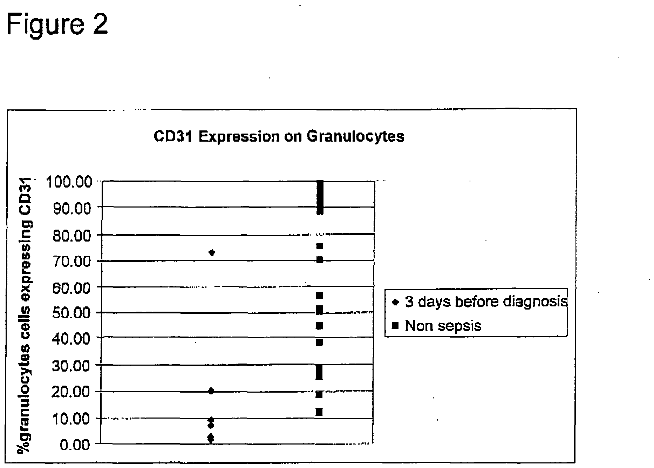

When the raw data for this is plotted (see Figure 2) it could be seen that 4 out of 6 (66.6 %) of the sepsis patients had CD 31 expression that was lower than that of the control group. It can be seen that the control group (non sepsis) data points are distributed between 11.8 % and 100 %, while four of the six data points in the three days before diagnosis measurements were less than 9 %. Therefore it is possible that CD31 may therefore be used to predict the onset of sepsis three days prior to the appearance of clinical signs and symptoms. This suggests that CD31 could be a useful predictive marker, particularly in combination with other informative sepsis biomarkers.

### Example 6: Multivariate statistical analysis to predict sepsis

### Introduction

Multivariate data analysis procedures were applied to data collected from patients 1-6 days prior to development of symptoms of Sepsis. Measurements included flow cytometry, PCR and classical clinical observations. Principle component analysis (PCA) was applied to the data matrix considering each of the three classes of observations individually and combined as a complete data set. Discriminant Function Analysis (DFA) was used to determine whether groups differ with regard to the mean of a variable, and then to use that variable to predict group membership (e.g., of new cases). This was performed on the results from PCA and on the three classes of observations individually and combined as a complete data set.

### Data description, manipulation and multivariate techniques

Prior to PCA, the data was summarised by producing probability density functions As normality of distribution is required prior to PCA and DFA, non-normal data were transformed using the Johnson transformation algorithm.

PCA is a dimensionality reducing technique which endeavours to decompose a multivariate data matrix into a few latent variables, composed of linear combinations of the variables, which explain the bulk of the variance of the original matrix. In this way correlations (positive or negative) of parameters within the data set can be established.

Essentially, DFA is similar in approach to Analysis of Variance (ANOVA). The DFA problem can be rephrased as a one-way analysis ANOVA problem. Specifically, one can ask whether two groups are significantly different from each other with respect to the mean of a particular variable. However, it should be clear that, if the means for a variable are significantly different in different groups, then it may be concluded that this variable discriminates between the groups.

In the case of a single variable, the final significance test of whether or not a variable discriminates between groups is the F-test. F is essentially computed as the ratio of the between-groups variance in the data over the pooled (average) within-group variance. If the between-group variance is significantly larger then there must be significant differences between means.

When considering multiple variables, it is possible to establish which of several variables contribute to the discrimination between groups. This results in a matrix of total variances and covariances; and likewise, a matrix of pooled within-group variances and covariances. These matrices are then compared via multivariate F-tests in order to determine whether or not there are any significant differences (with regard to all variables) between groups. This procedure is identical to multivariate analysis of variance or MANOVA. As in MANOVA, the multivariate test is performed, and, if statistically significant, which of the variables have significantly different means across the groups is examined. Thus, even though the computations with multiple variables are more complex, the principal reasoning still applies, namely, that variables that discriminate between groups are sought, as evident in observed mean differences.

DFA was performed on clinical, flow cytometry and RT-PCR data using the complete data matrix (including substituted mean values) and by exclusion of data points for which one or more parameters contained substituted mean values. Analogous models were developed to allow analysis of PCA scores from models developed in Model 1. The purpose of the latter was to establish if transformed data matrices (PCA) could be used to classify observations.

### Model 1. Principle Component Analysis (PCA) of observation data

### i) PCA model based on clinical data

The number of PCs derived and used in a given model is usually defined as those having an Eigenvalue of >1. 6 PCs meet this criterion for clinical data and explain a total of 74.3% of the variance of the data set. Since each of the PCs is orthogonal (uncorrelated) with respect to the other PCs, the association of a clinical parameter with a particular PC defines the PC and illustrates how the parameter influences the variance of the data set.

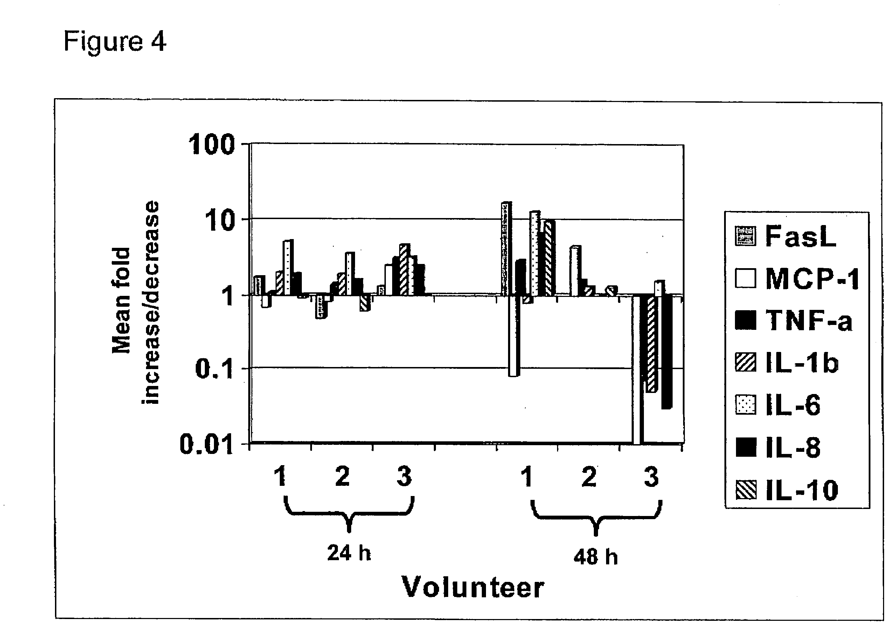

Table 12 summarises the loadings of each parameter with the six derived PCs from the clinical data. Loading values of >0.5 indicate a strong contribution of a particular parameter to a given PC. The PCs derived from the data set may be interpreted as follows:
- PC1: this is dominated by the strong correlation of WCC, monocytes, neutrophils and platelets. A strong correlation exists between creatinine and lactate. Both these groups have a negative relationship (opposite ends of PC1 scale) and are therefore negatively correlated. BXS and HCO₃⁻ are highly correlated and contribute to PC1 and PC2 equally. The latter parameters are contrasted by creatinine and lactate in PC1. These correlations are summarised in Table 2 and Figure 2
- PC2: shows a negative correlation between the group composed of WCC, monocytes, neutrophils and platelets and the group composed of BXS and HCO₃⁻
- PC3: this PC is characterised by the strong relationship between temp, HR and CRP as shown in Figure 3
- PC4: although many parameters approach significance for this PC, only CRP is definitively associated with this PC as demonstrated by Figure 4.
- PC5: pO₂ is contrasted with both urea and MAP in this component.
- PC6: this PC exclusively explains the variance introduced into the data set by lymphocytes.

In interpreting the PC loadings, correlated clinical parameters suggest levels of these species/physical parameters will be elevated or decreased in patients belonging characterised as belonging to PC1, 2 etc. This will be performed in the discriminant analysis section.

### ii) PCA model based on flow cytometry data

The parameters were abbreviated for clarity and abbreviations listed in Table 13. Nine PCs account for 80.5% of the variance of the data set as shown in the eigenvalue matrix in Table 14 and associated loadings are summarised in Table 15. The correlations between the measured parameters are shown in Table 16 with strong correlations within the derived PCs between the following:
fc 1 and fc 3
fc 5 and fc 6
fc 7 and fc 8
fc 9 with fc 10 and fc 11,
fc12andfc13
fc 11 and fc 14
fc 17 with fc 20 and fc 23
fc 21 with fc 17, 20 and 22
fc 23 with fc 20 and fc 22
fc 28 and fc 29

The PC structure may be interpreted as follows:
- PC1: correlates CD3 CD4 CD25 in CD3 CD4, CD3 CD4 HLA-DR in CD3 CD4, CD3 CD8 CD25 in CD3 CD8, CD19 CD80 in CD19, CD19 CD86 in CD19, CD14 CD80 in CD14, CD14 CD86 in CD14, CD19 CD54 in CD19, CD19 CD25 in CD19 and CD56 CD54 in CD56.
- PC2: contrasts CD19 CD86 in CD19 with CD14 HLA-DR in CD14, CD14 HLA-DR CD11B in CD14, CD14 HLA-DR CD11B CD54 in CD14.
- PC3: CD3 CD4 CD54 in CD3 CD4, CD3 CD4 CD69 in CD3 CD4, CD3 CD8 CD54 in CD3 CD8, CD3 CD8 CD69 in CD3 CD8
- PC4: CD56 CD69 in CD56, CD69 (%), CD11B CD69 (%)
- PC5: CD3 CD8 CD54 in CD3 CD8, CD62L (%)
- PC6: CD31 (%)
- PC7: no significant components with EV>0.5
- PC8: CD54 (%)
- PC9: CD14 CD11B in CD14

By considering only one parameter of a pair or group, it would be possible to remove 9 parameters thus increasing the y component of the data matrix. However, it was decided that CD31 (%), CD54 (%), CD62L (%), CD11B (%), CD69 (%) and CD11B CD69 (%) only be subjected to statistical analysis (fc 24-fc 29).

The eigenvalue matrix of the selected flow cytometry variables is shown in Table 17 and loadings of the PCA model constructed summarised in Table 18. The use of 3 PCs explains 76.6% of the variance of the data set. The PC model shows the following:
- PC1: correlates CD69 (%) and CD11B CD69 (%)
- PC2: correlates CD31 (%), CD62L (%) and CD11B (%)
- PC3: is composed of the variance associated with CD54 (%)

### iii) PCA model based on RT-PCR data

Table 19 indicates that 72.9% of the variance of the RT-PCR data is explained by only 3 PCs. The loading for this model are shown in Table120. The correlation of variables with each PC are shown in Figures 23 and 24 and reveal the following:
- PC1: correlates Fas-L, MCP-1, TNF-alpha, IL-6 and II-8
- PC2: correlates IL-1 and IL 10
- PC3: contrasts IL-1 and IL-10

The correlation of IL-1 and IL-10 in PC2 and subsequent contrast of these variables in PC3 is interesting. It appears that in some patients these vairables may be highly correlated or contrasted possibly providing a powerful means of discriminating patients.

### iv) PCA model based on combined clinical, flow cytometry and RT-PCR data

Table 21 summarises the parameters included in this final model and the associated Eigenvalues for the correlation matrix. Table 21 indicates that 9 PCs have an Eigenvalue greater than 1 which explain 68.7% of the data variance. The loadings of the model are shown in Table 22. Analysis allows the following interpretation of the PCA model:
- PC1: shows positive correlation between WCC, Neutrophils, Monocytes, APTR, HCO3-, BXS, Platelets, CD69 (%) and CD11B CD69 (%). This PC also contrasts the above with Lactate and Creatinine which are correlated.
- PC2: correlates CD69 (%), CD11B CD69 (%), WCC, Neutrophils, Monocytes and INR. These parameters are contrasted with TNF-alpha.
- PC3: strongly correlates the PCR parameters Fas-L, MCP-1, TNF-alpha, IL-6 and IL-8
- PC4: contrasts CRP and IL-10.
- PC5: correlates the flow cytometry parameters CD31 (%), CD54 (%),CD62L (%) and CD69 (%).
- PC6: correlates CD62L (%) and HR.
- PC7: is associated with Temp.
- PC8: is associated with IL-1
- PC9: is associated with PO2

### Model 2:_Discriminant Function Analysis (DFA) based on observations and PCA score data

The terminology common to all model definition in the DFA models developed is explained below and numerical values shown in Table 23.

### Model

The object of the analysis is to build a "model" of how to best predict to which group a case belongs. In the following discussion the term "in the model" will be used in order to refer to variables that are included in the prediction of group membership, and "not in the model" if they are not included.

### • Forward stepwise analysis

In stepwise discriminant function analysis, a model of discrimination is constructed step-by-step. Specifically, at each step all variables are reviewed and evaluated to establish which one will contribute most to the discrimination between groups. That variable will then be included in the model.

### • Backward stepwise analysis

It is possible to step backwards; in that case the programme first includes all variables in the model and then, at each step, eliminates the variable that contributes least to the prediction of group membership. Thus, as the result of a successful discriminant function analysis, one would only keep the "important" variables in the model, that is, those variables that contribute the most to the discrimination between groups.

### • F to enter, F to remove

The stepwise procedure is "guided" by the respective F to enter and F to remove values. The F value for a variable indicates its statistical significance in the discrimination between groups, that is, it is a measure of the extent to which a variable makes a unique contribution to the prediction of group membership. In general, the programme continues to choose variables to be included in the model, as long as the respective F values for those variables are larger than the user-specified F to enter; and excludes (removes) variables from the model if their significance is less than the user-specified F to remove.

### • Tolerance

The tolerance value of a variable is computed as 1-R² of the respective variable with all other variables in the model. Thus, the tolerance is a measure of the respective variable's redundancy. For example, a tolerance value of .10 means that the variable is 90% redundant with the other variables in the model.

### • Wilks' λ

This parameter gives a measure of the discriminatory power of the model and can assume values in the range of 0 (perfect discrimination) to 1 (no discrimination).

### • Partial λ

This is the Wilks' λ associated with the unique contribution (measured orthogonally) of the respective variable to the discriminatory power of the model.

As a point of note, a common misinterpretation of the results of stepwise discriminant analysis is to take statistical significance levels at face value. When the programme decides which variable to include or exclude in the next step of the analysis, it actually computes the significance of the contribution of each variable under consideration. Therefore, by nature, the stepwise procedures will capitalize on chance because they "pick and choose" the variables to be included in the model so as to yield maximum discrimination. Thus, when using the stepwise approach awareness that the significance levels do not reflect the true alpha error rate, that is, the probability of erroneously rejecting H0 (the null hypothesis that there is no discrimination between groups) must be maintained

### • Canonical Correlation Analysis (CCA)

This is an additional procedure for assessing the relationship between variables. Specifically, this manipulation allows the elucidation of the relationship between two sets of variables. Parameters which characterise this analysis are detailed below.

### • Significance of roots (χ² test)

The term root is used to describe the individual discriminant functions (DFs). The statistical significance of the derived DFs, is tested by the χ² test of successive DFs. A report of the step-down test of all canonical roots is obtained containing the significance of all DFs followed by the second line which reports the significance of the remaining roots, after removing the first root, and so on. Thus the number of DFs to interpret is obtained.

### • Discriminant function coefficients

Two outputs are produced, one for the Raw Coefficients and one for the Standardized Coefficients. Raw here means that the coefficients can be used in conjunction with the observed data to compute (raw) discriminant function scores. The standardized coefficients are the ones that are customarily used for interpretation, because they pertain to the standardized variables and therefore refer to comparable scales.

### • Eigenvalues

An Eigenvalue for each DF and the cumulative proportion of explained variance accounted for by each function is obtained. This value is defined in an identical way in PCA and DFA. The larger the value, the greater the amount of variance explained by that DF.

### • Factor structure coefficients

These coefficients represent the correlations between the variables and the DFs and are commonly used in order to interpret the "meaning" of discriminant functions. In an analogous way to PCA, the interpretation of factors should be based on the factor structure coefficients.

### • Means of canonical variables

When knowledge of how the variables participate in the discrimination between different groups is obtained, the next logical step is to determine the nature of the discrimination for each DF. The first step to answer this question is to look at the canonical means. The larger the canonical mean for a given DF and group of observations, the greater the discriminatory power of that DF.

### i) DFA model based on PCA scores of clinical data

### a) Containing substituted mean values

Table 19 summarises the results of this analysis. The Wilks' λ value of 0.4 indicates a relatively inefficient classification model. The three derived DFs account for a total of 89.9% of the variance of the data set and the DFs are composed mainly of PCs 1, 3 and 4. The factor structure coefficients indicate that:
- DF1 is composed of the variance explained by PC1 and to a lesser extent with PC4
- DF2 is exclusively composed of the variance explained by PC3
- DF3 is composed of the variance explained by PC4

These correlations are confirmed by the standardised coefficients. The means of cannonical variables indicate that:
- DF1 negatively correlates days 1, 2 and 3 with days 5 and 6
- DF2 defines control group observations
- DF3 defines observations for day 2

A summary of the classification of this model and its discriminative nature in relation to the PCs is shown in Table 25. The classification matrix for the model is shown in Table 26. Table 26 suggests a good classification can be obtained for control and 6 day data with 80 and 83 % respectively of observations being classified correctly. However the overall classification power of the model is poor with only 48 % of all observations being correctly classified.

### b) Excluding substituted mean values

Table 24 summarises the results of this analysis. The Wilks'λ value of 0.45 indicates a relatively inefficient classification model. The three derived DFs account for a total of 95 % of the variance of the data set and the DFs are composed mainly of PCs 1, 3 and 5. The factor structure coefficients indicate that:
- DF1 is composed of the variance explained by the negative correlation between PC1 and PC5
- DF2 is composed of the variance explained by PC3
- DF3 is composed of the variance explained by PC3 but to a lesser degree than DF2

These correlations are confirmed by the standardised coefficients. The means of cannonical variables indicate that:
- DF1 negatively correlates days 1, 2 and 3 with days 5 and 6
- DF2 negatively correlates days 1 and 6 with the control group
- DF3 defines observations for day 6

A summary of the classification of this model and its discriminative nature in relation to the PCs is shown in Table 25. The classification matrix for the model is shown in Table 26. Table 26 suggests a good classification can be obtained for control and 6 day data with 83 and 67 % respectively of observations being classified correctly. However the overall classification power of the model is poor with only 44 % of all observations being correctly classified, less than that using mean substituted variables.

The similar prediction efficiency with and without mean substituted values validated the subsequent approach to perform DFA with the inclusion of these values.

### ii) DFA model based on transformed values of clinical data

In an effort to improve the classification of observations, the transformed variable values from the original data matrix were subjected to DFA. The thesis was that since PCA is a dimensionality reducing technique, perhaps some data quality is lost and performing DFA on PCA scores leads to a model with less predictive power.

Table 27 summarises the results of this analysis. The Wilks' λ value of 0.22 is an improvement on the PCA scores classification models. The five derived DFs account for a total of 99 % of the variance of the data set and the DFs are composed of BXS, CRP, lactate, urea, temperature, creatinine, neutrophils, pO₂ and HCO₃⁻ with the other clinical variable having no influence on the classification of observations. The factor structure coefficients indicate that:
- DF1 classifies the correlation between BXS and HCO₃⁻ which are negatively correlated with lactate
- DF2 classifies observations showing a high degree of correlation between BXS, CRP and HCO₃⁻
- DF3 classifies samples with a negative correlation between temperature and creatinine
- DF4 classifies samples with a negative correlation between temperature and PO2
- DF5 classifies samples with a negative correlation between urea and neuts

A summary of the classification of this model and its discriminative nature in relation to the clinical variables is shown in Table 31. The classification matrix for the model is shown in Table 32. Table 32 suggests a good classification can be obtained for control and 6 day data with 80 and 83 % respectively of observations being classified correctly. Days 1, 2 and 5 are greatly improved compared to the PCA scores models but the overall classification power of the model is poor with 55 % of all observations being correctly classified.

### iii) DFA model based on PCA scores of flow cytometry

Table 33 summarises the results of this analysis. The Wilks' λ value of 0.39 indicates a relatively inefficient classification model. The two derived DFs account for a total of 71 % of the variance of the data set and the DFs are composed mainly of PCs 1, 5 and 5. The factor structure coefficients indicate that
- DF1 is composed of the variance explained by PC1 and PC8
- DF2 is exclusively composed of the variance explained by PC5

These correlations are confirmed by the standardised coefficients. The means of cannonical variables indicate that:
- DF1 negatively correlates day 1 with days 5 and 6
- DF2 defines day 3 observations

A summary of the Classification of this model and its discriminative nature in relation to the PCs is shown in Table 34. The classification matrix for the model is shown in Table 35. Table 35 suggests a reasonable classification can be obtained for control and 6 day data with 66% of observations being classified correctly in both groups. However the overall classification power of the model is poor with only 44 % of all observations being correctly classified.

### iv) DFA model based on flow cytometry data

A summary of the classification of this model and its discriminative nature in relation to the variables is shown in Table 36. The Wilks' λ value of 0.034 indicates an excellent classification model. The three derived DFs account for a total of 74% of the variance of the data set and the DFs are composed mainly of fc7-8, fc11, fc16, fc25, fc28, fc29. The factor structure coefficients indicate that:
- DF1 correlates fc7, 16, 28 and 29 and contrasts these to fc8 and 25
- DF2 correlates fc7, 8, 16 and 25 and contrasts these with fc12
- DF3 is correlated with fc11

Table 37 summarises this information.

These correlations are confirmed by the standardised coefficients. The means of cannonical variables indicate that:
- DF1 contrasts 1 day with days 5 and 6
- DF2 contrasts the control group with days 3, 4 and 5 and correlates the control with day 6
- DF3 contrasts days 2 and 3 with day 4

Table 38 suggests a good classification can be obtained all groups. The overall classification power of the model is impressive with 76.6% of all observations being correctly classified.

The DFA models for RT-PCR were so poor for both PCA scores and transformed data, with The Wilks' λ values >0.8, they were discarded and will not be considered further.

### v) DFA model based on combined clinical, flow cytometry and RT-PCR data

A summary of the classification of this model and its discriminative nature in relation to the variables is shown in Table 39. The Wilks' λ value of 0.0087 indicates an excellent classification model. The four derived DFs account for a total of 89.2% of the variance of the data set and the DF factor structure indicates that:BXS, fc 25, fc 22, fc 11, Temp, CRP, fc 18, fc 6, lL-6, INR, APTR, fc 16, Urea, Lactate, Fas-L, fc 13, fc 24, fc 1, fc 3, MCP-1, fc 28, II-10, fc 27, fc 26, Neutrophils, fc 14, WCC, fc 29, Platelets, pO₂ are included in the model. All other parameters fail to meet the stepwise criteria and hence were eliminated from the model.

The means of canonical variables indicate that:
- DF1 contrasts 1 day with days 5 and 6
- DF2 correlates the control group with day 6 and contrasts these with days 3, 4 and 5
- DF3 correlates the control group with day 5 and contrasts these to days 2, 3 and 6
- DF4 contrasts days 4 and 5

Table 40 shows an excellent classification can be obtained for all groups with a minimum correct assignment rate of 76%. The overall classification power of the model is impressive with 86.9% of all observations being correctly classified.

When each DF is applied to the data using this model, the groups of patients can clearly be seen to cluster and are spatially separated from the other groups.

### Conclusions

PCA has highlights correlations between measured variables for all classes of patients. Many of the correlations are expected from a molecular biology standpoint. Some of the PCA models greatly reduced the dimensionality of the data set but the resulting scores did not spatially separate the groups of patients.

DFA on scores obtained from PCA showed disappointing results. The discriminatory power of the models ranged from 44 - 56 % when PCA scores were used. The low discriminatory power of these models may be a result of the reduction in dimensionality of the data set during PCA with significant detail being lost. Using transformed variables in DFA gave much improved models. The discriminatory power of the clinical and flow cytometry models was 55 and 76 % respectively. When DFA was performed on the complete data set (clinical, flow cytometry and RT-PCR variables) a prediction efficiency of 86.9% was observed. Therefore it is recommended that the variables included in this latter model (Table 36) be measured and used to classify new patients suspected of being susceptible to sepsis.

The most impressive feature of the model is its ability to correctly assign patience correctly 6 days before the onset of symptoms. Therefore key discriminatory variables could be monitored and threshold levels established at which medical treatment must be administered. Using the parameters shown in Table 34 it is possible to acquire data from patients and using transformation algorithms input the data into the DFA model. This is then capable of classifying patients into the appropriate groups with an efficiency of approaching 90%. This could of great value when used in clinical laboratories.

**Table 11. Eigenvalues of correlation matrix, and related statistics for clinical observations**

| *PC* | *Eigenvalue* | *% Total variance* | *Cumulative Eigenvalue* | *Cumulative variance %* |
|---|---|---|---|---|
| 1 | 3.73 | 24.87 | 3.73 | 24.87 |
| 2 | 2.50 | 16.70 | 6.23 | 41.58 |
| 3 | 1.53 | 10.23 | 7.77 | 51.81 |
| 4 | 1.22 | 8.14 | 8.99 | 59.95 |
| 5 | 1.10 | 7.39 | 10.10 | 67.35 |
| 6 | 1.04 | 6.96 | 11.14 | 74.31 |
| 7 | 0.87 | 5.80 | 12.01 | 80.11 |
| 8 | 0.77 | 5.19 | 12.79 | 85.31 |
| 9 | 0.66 | 4.42 | 13.46 | 89.73 |
| 10 | 0.54 | 3.62 | 14.00 | 93.36 |
| 11 | 0.44 | 2.99 | 14.45 | 96.35 |
| 12 | 0.29 | 1.95 | 14.74 | 98.30 |
| 13 | 0.210 | 1.40 | 14.95 | 99.70 |
| 14 | 0.02 | 0.16 | 14.98 | 99.87 |
| 15 | 0.01 | 0.12 | 15.00 | 100.00 |

**Table 12. Loadings of clinical measurements for each PC. (associations with Eigenvalues >0.5 shown for 95% CL).**

| *Clinical measurement* | *PC1* | *PC 2* | *PC3* | *PC 4* | *PC 5* | *PC 6* |
|---|---|---|---|---|---|---|
| Temp | 0.14 | 0.08 | 0.60 | -0.36 | 0.14 | 0.40 |
| HR | 0,15 | -0.06 | 0.58 | -0.48 | 0.11 | -0.37 |
| MAP | 0.22 | 0.21 | -0.29 | -0.48 | -0.54 | -0.14 |
| WCC | 0.63 | -0.74 | 0.00 | 0.05 | 0.02 | -0.01 |
| Neuts | 0.58 | -0.75 | 0.01 | 0.17 | 0.00 | 0.05 |
| Lymphocytes | 0.35 | 0.00 | -0.14 | -0.16 | -0.19 | -0.62 |
| Monocytes | 0.62 | -0.66 | 0.08 | -0.05 | 0.01 | -0.01 |
| platelets | 0.53 | -0.09 | -0.37 | 0.05 | 0.22 | 0.04 |
| CRP | -0.15 | 0.11 | 0.62 | 0.50 | 0.08 | -0.36 |
| PO2 | -0.26 | 0.07 | -0.36 | 0.12 | 0.53 | -0.39 |
| HCO3- | 0.76 | 0.47 | 0.16 | 0.23 | -0.13 | -0.07 |
| BXS | 0.78 | 0.49 | 0-15 | 0.20 | -0.11 | -0.05 |
| Lactate | -0.55 | -0.46 | 0.16 | -0.12 | -0.08 | -0.22 |
| Urea | -0.30 | -0.17 | 0.09 | 0.44 | -0.58 | 0.04 |
| Creatinine | -0.69 | -0.44 | 0.05 | 0.02 | -0.19 | -0.06 |

**Table 13. Abbreviations used in analysis of flow cytometry data**

| *Abbreviation* | *flow cytometry parameter* |
|---|---|
| fc 1 | CD3 CD4 CD25 in CD3 CD4 |
| fc 2 | CD3 CD4 HLA-DR in CD3 CD4 |
| fc 3 | CD3 CD8 CD25 in CD3 GD8 |
| fc 4 | CD3 CD8 HLA-DR in CD3 CD8 |
| fc 5 | CD3 CD4 CD54 in CD3 CD4 |
| fc 6 | CD3 CD4 CD69 in CD3 CD4 |
| fc 7 | CD3 CD8 CD54 in CD3 CD8 |
| fc 8 | CD3 CD8 CD69 in CD3 CD8 |
| fc 9 | CD19 CD80 in CD19 |
| fc 10 | CD19 CD86 in CD19 |
| fc 11 | CD14 CD80 in CD14 |
| fc 12 | CD14 CD86 in CD14 |
| fc 13 | CD19 CD54 in CD19 |
| fc 14 | CD19 CD25 in CD19 |
| fc 15 | CD56 CD54 in CD56 |
| fc 16 | CD56 CD69 in CD56 |
| fc 17 | CD14 CD54 in CD14 |
| fc 18 | CD14 HLA-DR in CD14 |
| fc 19 | CD14 CD11B in CD14 |
| fc 20 | CD14 CD54 HLA-DR in CD14 |
| fc 21 | CD14 HLA-DR CD11B in CD14 |
| fc 22 | CD14 HLA-DR CD11B CD54 in CD14 |
| fc 23 | CD14 CD11B CD54 in CD14 |
| fc 24 | CD31 (%) |
| fc 25 | CD54 (%) |
| fc 26 | CD62L (%) |
| fc27 | CD11B (%) |
| fc 28 | CD69 (%) |
| fc 29 | CD11B CD69 (%) |

**Table 14. Eigenvalues of correlation matrix, and related statistics for flow cytometry data**

| PC | Eigenvalue | % Total variance | Cumulative Eigenvalue | Cumulative variance % |
|---|---|---|---|---|
| 1 | 7.16 | 24.68 | 7.16 | 24.68 |
| 2 | 3.81 | 13.12 | 10.96 | 37.80 |
| 3 | 2.63 | 9.07 | 13.59 | 46.88 |
| 4 | 2.43 | 8.39 | 16.03 | 55.27 |
| 5 | 1.90 | 6.54 | 17.93 | 61.81 |
| 6 | 1.62 | 5.59 | 19.55 | 67.41 |
| 7 | 1.46 | 5.05 | 21.01 | 72.45 |
| 8 | 1.21 | 4.19 | 22.23 | 76.64 |
| 9 | 1.10 | 3.78 | 23.32 | 80.42 |
| 10 | 0.87 | 3.00 | 24.19 | 83.42 |
| 11 | 0.85 | 2.93 | 25.04 | 86.36 |
| 12 | 0.71 | 2.44 | 25.75 | 88.80 |
| 13 | 0.63 | 2.17 | 26.38 | 90.97 |
| 14 | 0.53 | 1.82 | 26.91 | 92.78 |
| 15 | 0.39 | 1.36 | 27.30 | 94.14 |
| 16 | 0.35 | 1.21 | 27.65 | 95.35 |
| 17 | 0.34 | 1.16 | 27.99 | 96.51 |
| 18 | 0.31 | 1.07 | 28.30 | 97.58 |
| 19 | 0.20 | 0.68 | 28.50 | 98.26 |
| 20 | 0.12 | 0.42 | 28.62 | 98.68 |
| 21 | 0.10 | 0.34 | 28.72 | 99.02 |
| 22 | 0.08 | 0.27 | 28.79 | 99.29 |
| 23 | 0.07 | 0.23 | 28.86 | 99.52 |
| 24 | 0.05 | 0.18 | 28.91 | 99.70 |
| 25 | 0.04 | 0.13 | 28.95 | 99.83 |
| 126 | 0.02 | 0.08 | 28.98 | 99.91 |
| 27 | 0.01 | 0.05 | 28.99 | 99.96 |
| 28 | 0.01 | 0.03 | 29.00 | 99.99 |
| 29 | 0.00 | 0.01 | 29.00 | 100.00 |

**Table 15. Loadings for all flow cytometry data for each PC. (associations with Eigenvalues >0.5 shown for 95% CL)**

| | PC1 | PC2 | PC3 | PC4 | PC5 | PC6 | PC7 | PC8 | PC9 |
|---|---|---|---|---|---|---|---|---|---|
| fc 1 | **0.59** | -0.36 | 0.19 | 0.06 | -0.13 | -0.28 | 0.28 | -0.11 | -0.33 |
| fc 2 | **0.61** | -0.07 | -0.20 | -0.21 | 0.11 | 0.41 | 0.34 | -0.01 | -0.17 |
| fc 3 | **0.62** | -0.31 | -0.07 | 0.14 | 0.14 | -0.11 | 0.21 | 0.05 | -0.31 |
| fc 4 | 0.24 | -0.03 | -0.26 | -0.19 | 0.11 | 0.44 | 0.46 | -0.24 | -0.03 |
| fc 5 | -0.06 | -0.01 | **0.89** | -0.24 | -0.10 | 0.07 | 0.20 | 0.07 | -0.10 |
| fc 6 | -0.11 | -0.11 | **0.62** | -0.02 | -0.45 | 0.06 | 0.42 | 0.15 | -0.07 |
| fc 7 | 0.08 | 0.18 | **0.64** | -0.39 | **0.50** | 0.05 | -0.25 | -0.09 | -0.06 |
| fc 8 | 0.14 | 0.29 | **0.63** | -0.41 | 0.43 | 0.03 | -0.24 | -0.12 | -0.07 |
| fc 9 | **0.70** | -0.52 | 0.17 | 0.05 | -0.15 | -0.01 | -0.05 | -0.03 | 0.18 |
| fc 10 | **0.58** | **-0.57** | 0.05 | 0.14 | -0.01 | -0.06 | -0.25 | -0.35 | 0.11 |
| fc 11 | **0.79** | -0.35 | -0.06 | -0.09 | 0.02 | 0.30 | 0.04 | 0.03 | 0.14 |
| fc 12 | **0.53** | -0.36 | 0.06 | 0.15 | -0.22 | -0.09 | -0.33 | -0.44 | 0.02 |
| fc 13 | **0.57** | -0.27 | 0.23 | -0.09 | 0.17 | -0.06 | 0.01 | -0.15 | 0.24 |
| fc 14 | **0.74** | -0.44 | -0.01 | 0.03 | 0.09 | 0.13 | 0.00 | 0.22 | 0.16 |
| fc 15 | **0.54** | -0.19 | 0.11 | 0.18 | 0.08 | 0.13 | -0.30 | 0.46 | 0.14 |
| fc 16 | 0.31 | -0.09 | -0.21 | **-0.63** | -0.07 | 0.45 | -0.07 | -0.03 | -0.10 |
| fc 17 | **0.73** | 0.26 | 0.02 | 0.26 | 0.03 | -0.15 | -0.13 | 0.19 | -0.28 |
| fc its | 0.49 | **0.64** | -0.02 | -0.10 | -0.39 | 0.23 | -0.15 | -0.06 | -0.07 |
| fc 19 | 0.28 | 0.28 | 0.00 | -0.27 | -0-01 | -0.32 | 0.40 | 0.01 | **0.60** |
| fc 20 | **0.64** | **0.64** | -0.05 | 0.09 | -0.18 | 0.07 | -0.16 | 0.01 | -0.19 |
| fc 21 | **0.52** | **0.65** | 0.01 | -0.15 | -0.38 | 0.08 | -0.02 | -0.07 | 0.16 |
| fc 22 | **0.66** | **0.66** | -0.02 | 0.01 | -0.19 | -0.07 | -0.03 | -0.01 | 0.06 |
| fc 23 | **0.79** | 0.31 | 0.05 | 0.16 | 0.01 | -0.29 | 0.02 | 0.15 | 0.05 |
| fc 24 | 0.45 | 0.19 | -0.05 | -0.21 | 0.23 | **-0.53** | 0-33 | -0.02 | -0.04 |
| fc 25 | 0.29 | -0.15 | -0.12 | 0.00 | 0.29 | 0.03 | -0.01 | **0.54** | -0.12 |
| fc 26 | 0.10 | 0.35 | -0.24 | -0.03 | 0.55 | 0.12 | 0.02 | 0.04 | 0.24 |
| fc 27 | 0.25 | 0.16 | -0.37 | -0.17 | 0.35 | -0.29 | 0.03 | -0.29 | -0.27 |
| fc 28 | -0.08 | -0.30 | -0.25 | **-0.77** | -0.27 | -0.26 | -0.19 | 0.16 | -0.05 |
| fc 29 | -0.03 | -0.29 | -0.26 | **-0.77** | -0.26 | -0.29 | -0.17 | 0.17 | -0.04 |

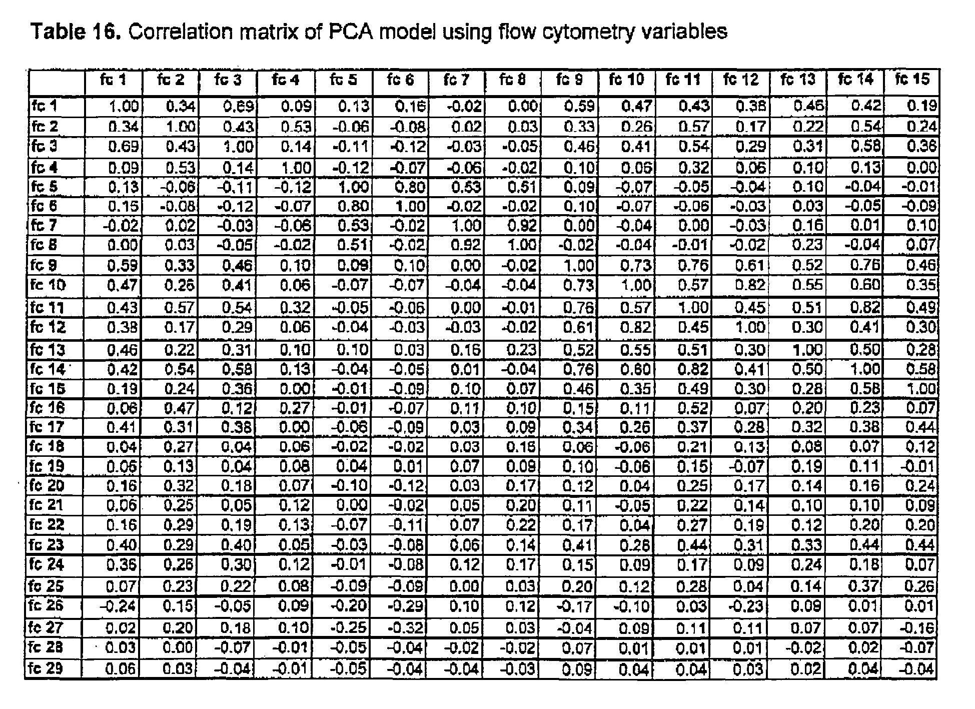

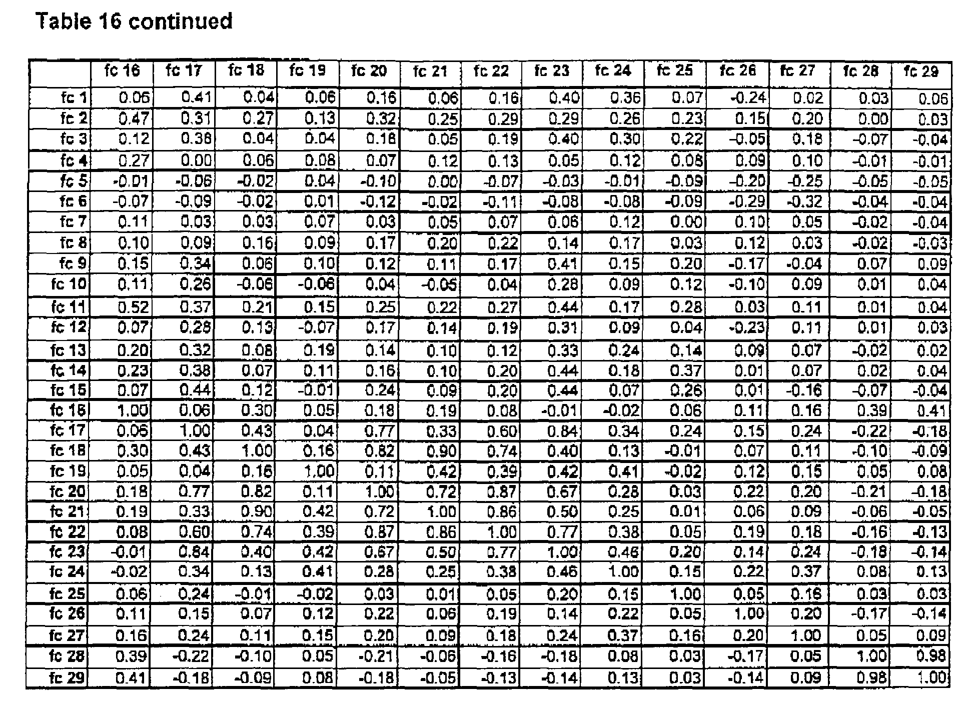

**Table 17. Eigenvalues of correlation matrix, and related statistics for selected flow cytometry variables (fc 24-fc 29)**

| *PC* | *Eigenvalue* | *% Total variance* | *Cumulative Eigenvalue* | *Cumulative variance %* |
|---|---|---|---|---|
| 1 | 2.06 | 34.26 | 2.06 | 34.26 |
| 2 | 1.60 | 26.64 | 3.65 | 60.90 |
| 3 | 0.94 | 15.71 | 4.60 | 76.60 |
| 4 | 0.76 | 12.70 | 5.36 | 89.31 |
| 5 | 0.63 | 10.45 | 5.99 | 99.76 |
| 6 | 0.01 | 0.24 | 6.00 | 100.00 |

**Table 18. Loadings for selected flow cytometry data for each PC. (associations with Eigenvalues >0.5 shown for 95% CL)**

| | PC1 | PC2 | PC3 |
|---|---|---|---|
| fc 24 | -0.23 | **-0.72** | -0.13 |
| fc 25 | -0.10 | -0.40 | **0.87** |
| fc 26 | 0.20 | **-0.60** | -6.39 |
| fc 27 | -0.19 | **-0.72** | -0.06 |
| fc 28 | **-0.98** | 0.14 | -0.05 |
| fc 29 | **-0.98** | 0.09 | -0.08 |

**Table 19. Eigenvalues of correlation matrix, and related statistics for RT-PCR data**

| PC | Eigenvalue | % Total variance | Cumulative Eigenvalue | Cumulative variance % |
|---|---|---|---|---|
| 1 | 2.9 | 41.0 | 2.9 | 41.0 |
| 2 | 1.2 | 17.6 | 4.1 | 58.6 |
| 3 | 1.0 | 14.4 | 5.1 | 73.0 |
| 4 | 0.6 | 8.3 | 5.7 | 81.3 |
| 5 | 0.6 | 7.9 | 6.2 | 89.2 |
| 6 | 0.4 | 6.2 | 6.7 | 95.4 |
| 7 | 0.3 | 4.6 | 7.0 | 100.0 |

**Table 20. Loadings for RT-PCR data for each PC. (associations with Eigenvalues >0.5 shown for 95% CL)**

| | PC1 | PC2 | PC3 |
|---|---|---|---|
| Fas-L | **0.68** | -0.01 | 0.340 |
| MCP-1 | **0.71** | -0.38 | -0.27 |
| TNF-alpha | **0.77** | 0.21 | -0.25 |
| IL-1 | 0.37 | **0.64** | **0.56** |
| IL-6 | **0.72** | -0.22 | -0.20 |
| IL-8 | **0.70** | -0.28 | 0.389 |
| IL-10 | 0.34 | **0.698** | **-0.48*** |

### • borderline significance at 95% CI

**Table 21. Eigenvalues of correlation matrix, and related statistics for combined clinical, RT-PCR and flow cytometry variables**

| | Eigenvalue | % Total variance | Cumulative Eigenvalue | Cumulative % |
|---|---|---|---|---|
| 1 | 4.550 | 15.167 | 4.550 | 15.167 |
| 2 | 3.861 | 12.870 | 8.411 | 28.037 |
| 3 | 3.295 | 10.982 | 11.706 | 39.019 |
| 4 | 2.028 | 6.760 | 13.734 | 45.779 |
| 5 | 1.816 | 6.052 | 15.549 | 51.831 |
| 6 | 1.520 | 5.065 | 17.069 | 56.896 |
| 7 | 1.234 | 4.113 | 18.303 | 61.010 |
| 8 | 1.153 | 3.844 | 19.456 | 64.854 |
| 9 | 1.139 | 3.797 | 20.595 | 68.650 |
| 10 | 0.958 | 3.192 | 21.553 | 71.842 |
| 11 | 0.931 | 3.104 | 22.484 | 74.946 |
| 12 | 0.906 | 3.021 | 23.390 | 77.968 |
| 13 | 0.825 | 2.748 | 24.215 | 80.716 |
| 14 | 0.740 | 2.466 | 24.955 | 83.182 |
| 15 | 0.685 | 2.284 | 25.640 | 85.466 |
| 16 | 0.611 | 2.036 | 26.251 | 87.502 |
| 17 | 0.559 | 1.863 | 26.810 | 89.365 |
| 18 | 0.542 | 1.808 | 27.352 | 91.173 |
| 19 | 0.480 | 1.601 | 27.832 | 92.774 |
| 20 | 0.441 | 1.469 | 28.273 | 94.244 |
| 21 | 0.371 | 1.235 | 28.644 | 95.479 |
| 22 | 0.333 | 1.108 | 28.976 | 96.588 |
| 23 | 0.299 | 0.998 | 29.276 | 97.586 |
| 24 | 0.250 | 0.833 | 29.526 | 98.419 |
| 25 | 0.175 | 0.585 | 29.701 | 99.003 |
| 26 | 0.139 | 0.462 | 29.840 | 99.465 |
| 27 | 0.114 | 0.381 | 29.954 | 99.846 |
| 28 | 0.020 | 0.068 | 29.974 | 99.914 |
| 29 | 0.018 | 0.059 | 29.992 | 99.973 |
| 30 | 0.008 | 0.027 | 30.000 | 100.000 |

**Table 22. Loadings for combined clinical, RT-PCR and flow cytometry data for each PC. (associations with Eigenvalues >0.5 shown for 95% CL)**

| parameter | PC1 | PC2 | PC3 | PC4 | PC5 | PC6 | PC7 | PC8 | PC9 |
|---|---|---|---|---|---|---|---|---|---|
| CD31 (%) | -0.02 | -0.03 | 0.42 | 0.18 | 0.52 | -0.07 | -0.40 | -0.17 | 0.03 |
| CD54 (%) | -0.16 | 0.04 | 0.20 | -0.09 | 0.48 | 0.48 | 0.06 | 0.18 | -0.19 |
| CD62L (%) | -0.41 | -0.09 | 0.20 | 0.28 | 0.16 | -0.21 | 0.01 | -0.01 | 0.39 |
| CD11B (%) | -0.13 | -0.16 | 0.22 | 0.19 | 0.54 | -0.13 | -0.09 | -0.19 | 0.05 |
| CD69 (%) | 0.49 | -0.55 | 0.23 | -0.27 | 0.13 | -0.16 | 0.00 | 0.40 | -0.25 |
| CD11B CD69 (%) | 0.48 | -0.53 | 0.29 | -0.27 | 0.14 | -0.16. | -0.03 | 0.40 | -0.22 |
| Temp | 0.20 | 0.06 | 0.30 | -0.35 | -0.02 | 0.37 | 0.47 | 0.17 | 0.30 |
| HR | 0.15 | -0.12 | 0.13 | -0.35 | 0.20 | 0.65 | -0.03 | -0.32 | 0.00 |
| MAP | 0.19 | 0.19 | -0.15 | 0.10 | -0.44 | 0.31 | -0.30 | 0.04 | -0.35 |
| WCC | 0.57 | -0.64 | 0.14 | 0.38 | 0.01 | 0.06 | 0.02 | -0.08 | 0.08 |
| Neuts | 0.53 | -0.64 | 0.21 | 0.38 | -0.01 | -0.05 | 0.00 | -0.06 | 0.12 |
| Lymphocytes | 0.31 | -0.10 | -0.32 | 0.01 | -0.02 | 0.09 | -0.06 | -0.30 | -0.28 |
| Monocytes | 0.56 | -0.61 | 0.04 | 0.25 | -0.04 | 0.19 | 0.06 | -0.04 | 0.09 |
| Platelets | 0.49 | -0.02 | -0.31 | 0.29 | 0.01 | -0.15 | 0.26 | 0.04 | -0.01 |
| INR | -0.21 | -0.71 | 0.09 | 0.10 | -0.25 | 0.21 | -0.24 | -0.12 | -0.04 |
| APTR | -0.50 | -0.49 | -0.04 | 0.07 | -0.33 | 0.11 | -0.22 | -0.08 | -0.08 |
| CRP | -0.07 | -0.09 | 0.26 | -0.63 | 0.14 | -0.32 | -0.23 | -0.18 | 0.02 |
| PO2 | -0.23 | 0.13 | -0.10 | 0.18 | 0.45 | -0.10 | 0.14 | -0.12 | -0.49 |
| HCO3- | 0.79 | 0.32 | -0.07 | -0.25 | -0.03 | -0.12 | -0.21 | -0.06 | 0.01 |
| BXS | 0.80 | 0.33 | -0.07 | -0.24 | -0.04 | -0.11 | -0.22 | -0.08 | 0.03 |
| Lactate | -0.50 | -0.41 | -0.02 | -0.15 | 0.03 | 0.06 | 0.22 | 0.08 | -0.08 |
| Urea | -0.25 | -0.15 | 0.21 | -0.12 | -0.24 | -0.32 | 0.16 | -0.08 | -0.19 |
| Creatinine | -0.68 | -0.45 | 0.16 | -0.06 | -0.15 | -0.04 | -0.03 | 0.10 | -0.18 |
| Fas-L | -0.05 | 0.12 | 0.77 | -0.08 | 0.01 | 0.11 | -0.16 | -0.05 | -0.05 |
| MCP-1 | 0.12 | 0.19 | 0.61 | 0.07 | -0.36 | -0.09 | 0.20 | -0.32 | -0.13 |
| TNF-alpha | 0.14 | 0.49 | 0.57 | 0.32 | 0.01 | 0.24 | 0.13 | 0.05 | -0.12 |
| IL-1 | -0.22 | 0.28 | 0.20 | 0.20 | -0.12 | 0.11 | -0.45 | 0.49 | 0.16 |
| IL-6 | 0.07 | 0.19 | 0.66 | 0.15 | -0.15 | -0.20 | 0.20 | -0.05 | -0.25 |
| IL-8 | 0.02 | 0.16 | 0.73 | -0.05 | -0.27 | -0.04 | -0.03 | -0.01 | 0.16 |
| IL-10 | 0.14 | 0.44 | 0.02 | 0.51 | 0.09 | 0.03 | -0.05 | 0.21 | -0.19 |

**Table 23. Model definition used in all DFA models**

| parameter | value |
|---|---|
| variable introduction | forward stepwise |
| tolerance | 0.010 |
| terms included in model | as derived |
| F to enter | 1.00 |
| F to remove | 0.00 |
| number of steps | as significant |

**Table 24. Summary of DFA model based on PCA scores of clinical data containing substituted mean values**

| parameter | | value/term | | |
|---|---|---|---|---|
| Wilks' λ | | < 0.40 | | |
| variables included in model | | PC1, PC3, PC4 | | |
| χ² | | 3 sig. DFs | | |

| cumulative proportion | | DF1 | DF2 | DF3 |
|---|---|---|---|---|
| | | 56.7% | 79.3% | 89.9% |
| Eigenvalue | | 0.60 | 0.24 | 0.11 |
| factor structure | PC1 | 0.63 | 0.02 | 0.15 |
| | PC3 | 0.09 | -0.87 | 0.39 |
| | PC4 | 0.44 | 0.03 | -0.51 |
| standardised coefficients | PC1 | **0.78** | 0.02 | 0.13 |
| | PC3 | 0.12 | **-0.89** | 0.35 |
| | PC4 | **0.61** | 0.04 | **-0.48** |
| means of cannonical variables | control | -0.15 | 0.63 | 0.04 |
| | day 1 | 0.51 | -0.39 | 0.02 |
| | day 2 | 0.57 | -0.17 | -0.64 |
| | day 3 | 0.72 | 0.02 | 0.35 |
| | day 4 | 0.21 | 0.02 | 0.43 |
| | day 5 | -1.80 | -0.01 | -0.28 |
| | day 6 | -1.33 | -1.14 | 0.37 |

**Table 25. summary of variable association in the discriminative DFA model based on PCA scores of clinical data containing substituted mean values**

| DF | PC | components of each PC |
|---|---|---|
| 1 | 1 | WCC, monocytes, neuts and platelets, BXS and HCO₃⁻-ve corr. with creatinine and lactate |
| | 4 | CRP |
| 2 | 3 | HR and CRP |
| 3 | 4 | CRP |

**Table 26. Classification matrix of DFA model based on PCA scores of clinical data containing substituted mean values**

| group | percent correct | control | 1 day | 2 days | 3 days | 4 days | 5 days | 6 days |
|---|---|---|---|---|---|---|---|---|
| | | p=.28 | p=.23 | p=.14 | p=.08 | p=.11 | p=.09 | p=.05 |
| control | 80 | 24 | 4 | 0 | 1 | 0 | 0 | 1 |
| 1 day | 36 | 12 | 9 | 3 | 0 | 0 | 0 | 1 |
| 2 days | 40 | 5 | 2 | 6 | 0 | 1 | 0 | 1 |
| 3 days | 11 | 1 | 5 | 1 | 1 | 1 | 0 | 0 |
| 4 days | 8 | 7 | 3 | 0 | 0 | 1 | 1 | 0 |
| 5 days | 50 | 2 | 2 | 0 | 0 | 0 | 5 | 1 |
| 6 days | 83 | 1 | 0 | 0 | 0 | 0 | 0 | 5 |
| Total | 48 | 52 | 25 | 10 | 2 | 3 | 6 | 9 |

**Table 27. Summary of DFA model based on PCA scores of clinical data without substituted mean values**

| parameter | | value/term | | |
|---|---|---|---|---|
| Wilks' λ | | < 0.45 | | |
| variables included in model | | PC1, PC3, PC5 | | |
| χ² | | 3 sig. DFs | | |

| cumulative proportion | | DF1 | DF2 | DF3 |
|---|---|---|---|---|
| | | 52% | 89% | 95% |
| Eigenvalue | | 0.49 | 0.35 | 0.05 |
| factor structure | PC1 | **0.65** | 0.09 | 0.08 |
| | PC3 | -0.13 | **0.80** | **0.45** |
| | PC5 | **-0.61** | -0.32 | -0.07 |
| standardised coefficients | PC1 | 0.79 | 0.10 | 0.06 |
| | PC3 | -0.15 | 0.87 | 0.38 |
| | PC5 | -0.73 | -0.34 | -0.06 |
| means of cannonical variables | control | -0.18 | **0.56** | -0.10 |
| | dav 1 | **0.54** | **-0.59** | 0.07 |
| | day 2 | **0.45** | -0.14 | 0.36 |
| | day 3 | **0.81** | 0.40 | -0.19 |
| | day 4 | 0.22 | -0.05 | -0.27 |
| | day 5 | **-1.61** | -0.00 | 0.33 |
| | day 6 | **-1.40** | **-1.90** | **-0.59** |

**Table 28. summary of variable association in the discriminative DFA model based on PCA scores of clinical data without substituted mean values**

| DF | PC | components of each PC |
|---|---|---|
| 1 | 1 | WCC, monocytes, neuts and platelets, BXS and HCO₃⁻-ve corr. with creatinine and lactate |
| | 5 | PO2 is contrasted with both urea and MAP |
| 2 | 3 | HR and CRP |
| 3 | 3 | HR and CRP |

**Table 29. Classification matrix of DFA model based on PCA scores of clinical data without substituted mean values**

| group | percent correct | control | 1 day | 2 days | 3 days | 4 days | 5 days | 6 days |
|---|---|---|---|---|---|---|---|---|
| | | p=.34 | p=.21 | p=.13 | p=.06 | p=.10 | p=.09 | p=.03 |
| control | 83 | 25 | 2 | 1 | 1 | 0 | 1 | 0 |
| 1 day | 47 | 9 | 9 | 1 | 0 | 0 | 0 | 0 |
| 2 days | 0 | 6 | 6 | 0 | 0 | 0 | 0 | 0 |
| 3 days | 0 | 3 | 3 | 0 | 0 | 0 | 0 | 0 |
| 4 days | 0 | 5 | 3 | 0 | 0 | 0 | 1 | 0 |
| 5 days | 25 | 4 | 2 | 0 | 0 | 0 | 2 | 0 |
| 6 days | 67 | 0 | 0 | 0 | 0 | 0 | 1 | 2 |
| Total | 44 | 52 | 25 | 2 | 1 | 0 | 5 | 2 |

**Table 30. Summary of DFA model based on transformed clinical data with substituted mean values**

| parameter | | value/term | | | | |
|---|---|---|---|---|---|---|
| Wilks' λ | | 0.25 | | | | |
| variables included in model | | BXS, CRP, lactate, urea, temperature, creatinine, neuts, PO2, HCO₃⁻ | | | | |
| χ² | | 5 sig. DFs | | | | |

| cumulative proportion | | DF1 | DF2 | DF3 | DF4 | DF5 |
|---|---|---|---|---|---|---|
| | | 46% | 74% | 85% | 94% | 99% |
| Eigenvalue | | 0.78 | 0.47 | 0.18 | 0.15 | 0.09 |
| factor structure | BXS | **-0.62** | **0.58** | -0.09 | 0.21 | 0.22 |
| | CRP | 0.12 | **0.63** | 0.16 | -0.03 | -0.31 |
| | temp | 0.17 | 0.03 | **-0.56** | **0.62** | 0.11 |
| | lactate | **0.65** | 0.03 | 0.05 | 0.05 | 0.01 |
| | urea | -0.19 | -0.18 | -0.19 | 0.17 | **-0.57** |
| | creatinine | 0.42 | -0.26 | **0.51** | 0.20 | -0.19 |
| | HCO₃⁻ | **-0.54** | **0.67** | -0.13 | 0.19 | 0.16 |
| | PO2 | 0.16 | -0.05 | -0.04 | **-0.67** | -0.31 |
| | neuts | -0.14 | 0.05 | -0.24 | -0.18 | **0.70** |
| standardised coefficients | BXS | **-1.45** | **-0.78** | **0.84** | 0.09 | **1.00** |
| | CRP | 0.13 | 0.65 | 0.07 | -0.20 | -0.20 |
| | temp | 0.41 | -0.02 | **-0.64** | **0.53** | 0.02 |
| | lactate | **0.56** | 0.34 | -0.22 | -0.01 | 0.21 |
| | urea | -0.52 | -0.22 | **-0.51** | 0.09 | **-0.60** |
| | creatinine | 0.13 | -0.11 | **0.93** | **0.57** | 0.10 |
| | HCO₃⁻ | **1.02** | **1.45** | **-0.53** | 0.32 | **-0.88** |
| | PO2 | 0.19 | 0.07 | -0.28 | **-0.61** | -0.21 |
| | neuts | -0.10 | 0.01 | -0.30 | -0.32 | **0.67** |
| means of canonical variables | control | **-0.54** | **-0.90** | -0.10 | -0.14 | -0.07 |
| | day 1 | -0.22 | **0.53** | -0.35 | -0.03 | 0.40 |
| | day 2 | -0.19 | **0.84** | 0.20 | **-0.58** | -0.36 |
| | day 3 | -0.42 | **0.64** | 0.41 | **0.62** | -0.28 |
| | day 4 | -0.34 | -0.09 | 0.43 | **0.61** | 0.06 |
| | day 5 | **2.05** | -0.37 | **0.61** | -0.23 | 0.27 |
| | day 6 | **1.95** | 0.03 | **-1.05** | 0.47 | **-0.54** |

**Table 31. Summary of variable association in the DFA model based on clinical data with substituted mean values**

| DF | clinical variables defining DFs |
|---|---|
| 1 | BXS and HCO₃⁻ which are negatively correlated with lactate |
| 2 | correlation between BXS, CRP and HCO₃⁻ |
| 3 | negative correlation between temperature and creatinine |
| 4 | negative correlation between temperature and PO2 |
| 5 | negative correlation between urea and neutrophilss |

**Table 32. Classification matrix of DFA model based on clinical data with substituted mean values**

| group | percent correct | control | 1 day | 2 days | 3 days | 4 days | 5 days | 6 days |
|---|---|---|---|---|---|---|---|---|
| | | p=.28 | p=.23 | p=.14 | p=.08 | p=.11 | p=.09 | p=.05 |
| control | 80 | 24 | 5 | 1 | 0 | 0 | 0 | 0 |
| 1 day | 56 | 4 | 14 | 5 | 0 | 1 | 0 | 1 |
| 2 days | 60 | 2 | 2 | 9 | 1 | 0 | 0 | 1 |
| 3 days | 11 | 1 | 4 | 2 | 1 | 1 | 0 | 0 |
| 4 days | 8 | 7 | 4 | 0 | 0 | 1 | 0 | 0 |
| 5 days | 50 | 1 | 2 | 1 | 0 | 0 | 5 | 1 |
| 6 days | 83 | 0 | 0 | 0 | 0 | 0 | 1 | 5 |
| Total | 55 | 39 | 31 | 18 | 2 | 3 | 6 | 8 |

**Table 33. Summary of DFA model based on PCA scores of flow cytometry data containing substituted mean values**

| parameter | | value/term | |
|---|---|---|---|
| Wilks' λ | | < 0.39 | |
| variables included in model | | PC1, PC5, PC8 | |
| χ² | | 2 sig. DFs | |

| cumulative proportion | | DF1 | DF2 |
|---|---|---|---|
| | | 46 % | 71 % |
| Eigenvalue | | 0.49 | 0.26 |
| factor structure | PC1 | 0.30 | -0.04 |
| | PC5 | -0.19- | **-0.59** |
| | PC8 | **0.419** | -0.14 |
| standardised coefficients | PC1 | **0.40** | -0.04 |
| | PC5 | -0.25 | **-0.67** |
| | PC8 | **0.56** | -0.16 |
| means of canonical variables | control | -0.21 | -0.35 |
| | day 1 | **-0.71** | -0.12 |
| | day 2 | -0.24 | 0.16 |
| | day 3 | 0.14 | **1.44** |
| | day 4 | 0.48 | 0.32 |
| | day 5 | **0.88** | -0.28 |
| | day 6 | **1.99** | -0.43 |

**Table 34. Summary of variable association in the DFA model based on PCA scores of flow cytometry data containing substituted mean values**

| DF | PC | components of each DF |
|---|---|---|
| 1 | 1 | fc 1-3, 9-15, 17, 20-23 |
| | 8 | fc 19 |
| 2 | 5 | fc 7 + 26 |

**Table 35 Classification matrix of DFA model based on PCA scores of flow cytometry data containing substituted mean values**

| group | percent correct | control | 1 day | 2 days | 3 days | 4 days | 5 days | 6 days |
|---|---|---|---|---|---|---|---|---|
| | | p=.28 | p=.23 | p=.14 | p=.08 | p=.11 | p=.09 | p=.05 |
| control | 66.6 | 20 | 7 | 0 | 1 | 0 | 1 | 1 |
| 1 day | 36.0 | 12 | 9 | 2 | 0 | 0 | 2 | 0 |
| 2 days | 13.3 | 4 | 8 | 2 | 1 | 0 | 0 | 0 |
| 3 days | 44.4 | 2 | 2 | 0 | 4 | 0 | 1 | 0 |
| 4 days | 33.3 | 3 | 2 | 1 | 0 | 4 | 1 | 1 |
| 5 days | 40.0 | 4 | 1 | 0 | 0 | 0 | 4 | 1 |
| 6 days | 66.6 | 1 | 1 | 0 | 0 | 0 | 0 | 4 |
| Total | 43.9 | 46 | 30 | 5 | 6 | 4 | 9 | 7 |

**Table 36 Summary of DFA model based on flow cytometry data containing substituted mean values**

| parameter | | value/term | | |
|---|---|---|---|---|
| Wilks' λ | | < 0.034 | | |
| variables included in model | | fc7-8, fc11, fc16, fc25, fc28, fc29 | | |
| χ² | | 3 sig. DFs | | |

| cumulative proportion | | DF1 | DF2 | DF3 |
|---|---|---|---|---|
| | | 46% | 62% | 74% |
| Eigenvalue | | 2.17 | 0.94 | 0.63 |
| factor structure | fc7 | **0.06** | **-0.16** | -0.10 |
| | fc8 | **-0.06** | **-0.19** | -0.07 |
| | fc11 | 0.02 | **0.13** | **-0.24** |
| | fc16 | **0.05** | **-0.14** | 0.14 |
| | fc25 | **-0.34** | **-0.23** | -0.08 |
| | fc28 | **0.04** | -0.02 | 0.02 |
| | fc29 | **0.06** | -0.03 | 0.01 |
| standardised coefficients | fc7 | 1.84 | 0.20 | -1.03 |
| | fc8 | -1.66 | -1.00 | 0.32 |
| | fc11 | 0.87 | 1.89 | -1.16 |
| | fc16 | -0.73 | -1.33 | 0.80 |
| | fc25 | -0.86 | -0.29 | 0.02 |
| | fc28 | -2.34 | 0.65 | 0.29 |
| | fc29 | 2.64 | 0.12 | -0.66 |
| means of canonical variables | control | 0.50 | **-0.92** | -0.40 |
| | day 1 | **1.07** | -0.32 | 0.12 |
| | day 2 | 0.22 | 0.07 | **1.10** |
| | day 3 | 0.20 | **1.75** | **1.26** |
| | day 4 | -0.39 | **1.49** | **-1.34** |
| | day 5 | **-1.05** | **0.73** | -0.55 |
| | day 6 | **-5.27** | **-1.06** | 0.48 |

**Table 37 Summary of variable association in the DFA model based on flow cytometry data containing substituted mean values**

| DF | Clinical variables defining DFs |
|---|---|
| 1 | correlates fc7, 16, 28 and 29 and contrasts these to fc8 and 25 |
| 2 | correlates fc7, 8, 16 and 25 and contrasts these with fc12 |
| 3 | correlated with fc11 |

**Table 38 Classification matrix of DFA model based on clinical data with substituted mean values**

| group | percent correct | control | 1 day | 2 days | 3 days | 4 days | 5 days | 6 days |
|---|---|---|---|---|---|---|---|---|
| | | p=.28 | p=.23 | p=.14 | p=.08 | p=.11 | p=.09 | p=.05 |
| control | 80.0 | 24.0 | 2.0 | 0.0 | 1.0 | 2.0 | 1.0 | 0.0 |
| 1 day | 68.0 | 6.0 | 17.0 | 1.0 | 0.0 | 1.0 | 0.0 | 0.0 |
| 2 days | 73.3 | 3.0 | 1.0 | 11.0 | 0.0 | 0.0 | 0.0 | 0.0 |
| 3 days | 66.7 | 2.0 | 0.0 | 0.0 | 60 | 1.0 | 0.0 | 0.0 |
| 4 days | 91.7 | 1.0 | 0.0 | 0.0 | 0.0 | 11.0 | 0.0 | 0.0 |
| 5 days | 80.0 | 0.0 | 1.0 | 1.0 | 0.0 | 0.0 | 8.0 | 0.0 |
| 6 days | 83.3 | 0.0 | 0.0 | 0.0 | 0.0 | 1.0 | 0.0 | 5.0 |
| Total | 76.6 | 36.0 | 21.0 | 13.0 | 7.0 | 16.0 | 9.0 | 5.0 |

**Table 39 Summary of DFA model based on combined clinical, RT-PCR and flow cytometry variables**

| parameter | | value/term | | | |
|---|---|---|---|---|---|
| Wilks' λ | | < 0.0087 | | | |
| variables included in model | | fc7-8, fc11, fc16, fc25, fc28, fc29 | | | |
| χ² | | 3 sig. DFs | | | |

| cumulative proportion | | DF1 | DF2 | DF3 | DF4 |
|---|---|---|---|---|---|
| | | 44% | 63% | 79% | 89% |
| Eigenvalue | | 3.79 | 1.60 | 1.34 | 0.86 |
| factor structure | BXS | **-0.30** | 0.08 | **-0.26** | **0.17** |
| | fc 25 | **0.22** | **-0.23** | -0.08 | **0.13** |
| | fc 22 | **0.22** | -0.03 | 0.08 | **0.28** |
| | fc 11 | -0.02 | **0.12** | 0.05 | **0.23** |
| | Temp | 0.09 | **-0.11** | **-0.13** | **0.20** |
| | CRP | 0.01 | **0.16** | **-0.33** | **-0.16** |
| | fc 18 | **0.15** | 0.01 | -0.12 | 0.06 |
| | fc 6 | -0.03 | 0.01 | -0.02 | **0.12** |
| | IL-6 | **0.11** | -0.06 | **0.11** | -0.07 |
| | INR | 0.00 | 0.06 | 0.03 | **0.12** |
| | APTR | **0.15** | **0.12** | **0.30** | -0.03 |
| | fc 16 | -0.07 | -0.09 | **-0.11** | -0.07 |
| | Urea | -0.02 | **-0.10** | 0.01 | **0.17** |
| | Lactate | **0.25** | 0.04 | -0.05 | **-0.22** |
| | Fas-L | 0.00 | **0.15** | -0.03 | 0.04 |
| | fc 13 | -0.04 | -0.01 | -0.07 | **0.11** |
| | fc 24 | 0.00 | **-0.15** | **0.11** | **0.15** |
| | fc 1 | -0.01 | **0.12** | **-0.12** | **0.22** |
| | fc 3 | -0.02 | 0.04 | -0.02 | **0.15** |
| | MCP-1 | -0.06 | 0.04 | 0.02 | -0.02 |
| | fc 28 | -0.05 | -0.03 | -0.08 | -0.01 |
| | Il-10 | 0.01 | **-0.10** | 0.09 | -0.07 |
| | fc 27 | 0.01 | **-0.12** | 0.08 | -0.02 |
| | fc 26 | 0.04 | **-0.10** | **0.19** | -0.06 |
| | Neuts | **-0.11** | -0.07 | 0.03 | -0.04 |
| | fc 14 | 0.00 | 0.09 | 0.08 | **0.19** |
| | WCC | **-0.10** | -0.08 | 0.03 | -0.01 |
| | fc 29 | -0.07 | -0.04 | -0.08 | 0.03 |
| | Platelets | **-0.11** | 0.04 | **0.12** | -0.03 |
| | PO2 | 0.05 | -0.07 | 0.03 | **-0.27** |

| parameter | | value/term | | | |
|---|---|---|---|---|---|
| cumulative proportion | | DF1 | DF2 | DF3 | DF4 |
| | | 44% | 63% | 79% | 89% |
| Eigenvalue | | 3.79 | 1.60 | 1.34 | 0.86 |
| standardised coefficients | BXS | -0.80 | 0.33 | -0.21 | -0.09 |
| | fc 25 | 0.81 | -0.52 | -0.29 | 0.22 |
| | fc 22 | 0.46 | -0.58 | 0.05 | 0.68 |
| | fc 11 | 0.04 | 2.00 | 0.53 | 0.96 |
| | Temp | 0.51 | -0.41 | -0.07 | 0.23 |
| | CRP | 0.07 | 0.14 | -0.58 | -0.13 |
| | fc 18 | 0.64 | 0.72 | -0.53 | -0.11 |
| | fc 6 | 0.02 | -0.29 | 0.17 | 0.08 |
| | IL-6 | -0.69 | -0.36 | 0.28 | -0.12 |
| | INR | -0.90 | 0.03 | -0.99 | 0.94 |
| | APTR | 0.82 | 0.62 | 1.28 | -0.44 |
| | fc 16 | -0.63 | -1.23 | -0.54 | -0.69 |
| | Urea | -0.13 | -0-38 | 0.06 | 0.58 |
| | Lactate | 0.14 | 0.12 | -0.06 | -0.68 |
| | Fas-L | 0.20 | 0.92 | -0.04 | 0.05 |
| | fc 13 | -0.27 | -0.82 | -0.33 | -0.07 |
| | fc 24 | -0.46 | -0.72 | 0.11 | 0.15 |
| | fc 1 | 1.04 | 0.84 | 0.09 | 0.12 |
| | fc 3 | -0.67 | -0-46 | -0.41 | 0.06 |
| | MCP-1 | 0.33 | 0.01 | -0.21 | -0.05 |
| | fc 28 | 3.20 | 0.89 | -2.48 | -0.21 |
| | Il-10 | 0.22 | -0-03 | 0.26 | -0.16 |
| | fc 27 | 0.12 | 0.14 | 0.23 | -0.13 |
| | fc 26 | 0.15 | 0.26 | 0.27 | -0.01 |
| | Neuts | 0.83 | 0.74 | 0.27 | -1.34 |
| | fc 14 | -0.43 | -0.73 | 0.32 | -0.88 |
| | WCC | -0.70 | -1.06 | 0.05 | 0.77 |
| | fc 29 | -2.73 | -0.34 | 2.71 | 0.56 |
| | Platelets | -0.47 | -0.06 | 0.33 | 0.27 |
| | PO2 | -0.17 | -0.12 | 0.20 | -0.45 |
| means af canonical variables | control | -0.391 | **-1.04** | **1.24** | 0.23 |
| | day 1 | **-1.80** | -0.51 | -0.50 | -0.19 |
| | day 2 | -0.65 | 0.70 | **-1.16** | -1.17 |
| | day 3 | -0.40 | **1.12** | **-1.19** | 0.45 |
| | day 4 | 0.38 | **2.02** | 0.20 | **1.77** |
| | day 5 | 3.33 | **1.36** | **1.41** | **-1.45** |
| | day 6 | 5.37 | **-2.44** | **-2.21** | 0.76 |

**Table 40. Classification matrix of DFA model based on combined clinical, RT-PCR and flow cytometry variables**

| group | percent correct | control | 1 day | 2 days | 3 days | 4 days | 5 days | 6 days |
|---|---|---|---|---|---|---|---|---|
| | | p=.28 | p=.23 | p=.14 | p=.08 | p=.11 | p=.09 | p=.05 |
| control | 90.0 | 27.0 | 0.0 | 1.0 | 1.0 | 1.0 | 0.0 | 0.0 |
| 1 day | 76.0 | 3.0 | 19.0 | 3.0 | 0.0 | 0.0 | 0.0 | 0.0 |
| 2 days | 93.3 | 0.0 | 1.0 | 14.0 | 0.0 | 0.0 | 0.0 | 0.0 |
| 3 days | 77.8 | 1.0 | 0.0 | 1.0 | 7.0 | 0.0 | 0.0 | 0.0 |
| 14 days | 91.7 | 0.0 | 0.0 | 1.0 | 0.0 | 11.0 | 0.0 | 0.0 |
| 5 days | 90.0 | 0.0 | 0.0 | 1.0 | 0.0 | 0.0 | 9.0 | 0.0 |
| 6 days | 100.0 | 0.0 | 0.0 | 0.0 | 0.0 | 0.0 | 0.0 | 6.0 |
| Total | 86.9 | 31.4 | 20.0 | 21.0 | 8.0 | 12.0 | 9.0 | 6.0 |

### Example 7: Binary logistic regression analysis to predict sepsis

A binary logistic regression model was used to analyse the RT-PCR, flow cytometry results and clinical data separately, from the ICU patients who went on to develop sepsis and presented positive microbiology results. This model used results gained from an age matched group of ICU patients who were not diagnosed with sepsis as the control group. Although the model identified numerous possible predictors some appeared to be of limited use since the values obtained for the pre-symptomatic sepsis patients were within those obtained for the non sepsis patients. The potential prediction markers that did yield some pre-sepsis data points that differed from the non sepsis data are listed in Table 36. However when combined, these prediction markers could only have identified 8 out of the 24 pre-sepsis patients.

**Table 41. Summary of potential prediction markers identified by binary logistic regression analysis.**

| **Time Point** | **Overall Predictor effect** | **Predictor** | **Probability score** |
|---|---|---|---|
| 5 days pre-diagnosis | p=0.10 | base excess | p=0.047 |
| 4 days pre-diagnosis | p=0.042 | IL-10 | P=0.058 |
| 3 days pre-diagnosis | p=0.095 | blood bicarbonate | P=0.021 |
| | p=0.114 | CD 31 | p=0.037 |
| | P=0.055 | IL-10 | p=0.052 |
| 2 days pre-diagnosis | p=0.10 | blood bicarbonate | p=0.017 |
| | | C reactive protein | p=0.038 |
| | P=0.085 | TNF-α | p=0.022 |

The discovery of a combination of markers that could possibly predict sepsis in 8 out of 24 patients who later went on to develop SIRS with confirmed infection dos not constitute a diagnostic test. Although the prediction capability for CD31 on granulocytes appeared promising (66%), this marker was only effective three days before the appearance of clinical symptoms. A test based on CD31 alone may not constitute a diagnostic test since to be effective there would need to be a larger diagnostic window. This could be achieved by the discovery of even more markers. This study may however have found some markers that could form part of a diagnostic test in the future, but caution must be exercised. In the mid 1980s HLA-DR was believed to be prognostic for the development of infections and sepsis (Spittler, A. & Roth, E. 2003, Intensive Care Med , vol. 29, pp. 1211-1213 More recent studies however have shown that post-operative levels of this marker did not predict the onset of SIRS, sepsis or infectious complications (Oczenski, W. et al 2003, Intensive Care Med, vol. 29, pp. 1253-1257 and Perry, S. et al. 2003, Intensive Care Med, vol. 29, pp. 1245-1252. The conflicting reports in the case of HLA-DR illustrates why caution must be applied to the results of this study. These findings could be due to regional factors such as antibiotic policy, diagnostic criteria, clinical practice, surgical procedures, treatment regimes, environmental factors and the patients predisposing factors (Angus, D. & Wax, R. 2001, Critical Care Medicine, vol. 29, no. 7 (suppi), pp. 109-116). A larger study that involves more patients from several different hospitals, preferably spanning different health authorities, needs to be conducted to further assess the usefulness of the markers identified for the prediction of sepsis.

### Example 8: Sepsis as a model for response to biological weapons

### Background and method

Since one of the applications for the claimed invention is the early detection of deliberate infection resulting from exposure to biological weapons, the applicability of sepsis as a model for such infection was examined. Presumptive biological weapons pathogens such as *Burkholderia pseudomallei* and *Francisella tularensis* are predicted to produce severe sepsis (see Table 42), which is difficult to model for obvious reasons.

However, *in vitro* infection of whole blood may be used as a model and the activation marker expression and cytokine response measured. To compare this *in vitro* infection model with the *in vivo* situation, *Staphylococcus aureus* infection was selected as a model infectious agent directly comparable with the *in vivo* hospital-acquired infection data.

**Table 42**

| **Infection** | **Forms of "Negative" Outcome** |
|---|---|
| Anthrax | Sepsis, septic shock |
| Brucellosis | Sepsis, septic shock or chronic form |
| Glanders and Melioidosis | Sepsis, septic shock or persistent form |
| Plague | Sepsis, septic shock |
| Tularemia | Sepsis, septic shock |
| Epidemic Typhus | Sepsis |
| Q fever | Sepsis |
| Ebola and Marburg hemorrhagic fevers | Sepsis, septic (toxico-infectious, hypovolemic) shock |
| Japanese encephalitis | Sepsis, septic shock |
| Smallpox | Sepsis, septic shock |
| Yellow fever | Sepsis |

Blood from 25 healthy volunteers was infected *in vitro* with *Staphylococcus aureus* and the following activation markers and cytokine levels measured at 24 and 48 hours post-infection, as previously described.

### FACS

Dendritic cells: CD54, CD97, CCR6, CCR7

NK cells: CD25, CD44, CD62L, CD69, CD97

Monocytes: CD44, CD54, CD62L, CD69, CD97, CD107a

Neutrophils, CD44, CD62L, CD69, CD107a

### Real time RT PCR

IL-1β, IL-6, IL-8, IL-10, MCP-1, TNF-α, sFasL

Each of these sets of input parameters (ie Dendritic cell markers, NK cell markers, monocyte markers, neutrophil markers, RT PCR data at 24h, RT PCR data at 48h) were used to train its own neural network model. Random selections of infected or non-infected blood samples were used for training (70%) or subsequent testing (30%). The testing phase of the neural network analysis gave a predictive accuracy based on the % of times it would correctly predict that the test set of input parameters was from an infected or non-infected sample. This testing of each set of input parameters was repeated 5 times. Each time the test was conducted a new neural network was constructed using a newly randomised 70% of the infected and non-infected samples. An average predictive accuracy was derived for each set of input parameters by working out the mean from the 5 predictive accuracies calculated from the 5 neural networks constructed on the 5 randomised sets of input data. The methodology was similar to that used in the sepsis patient study.

### Results

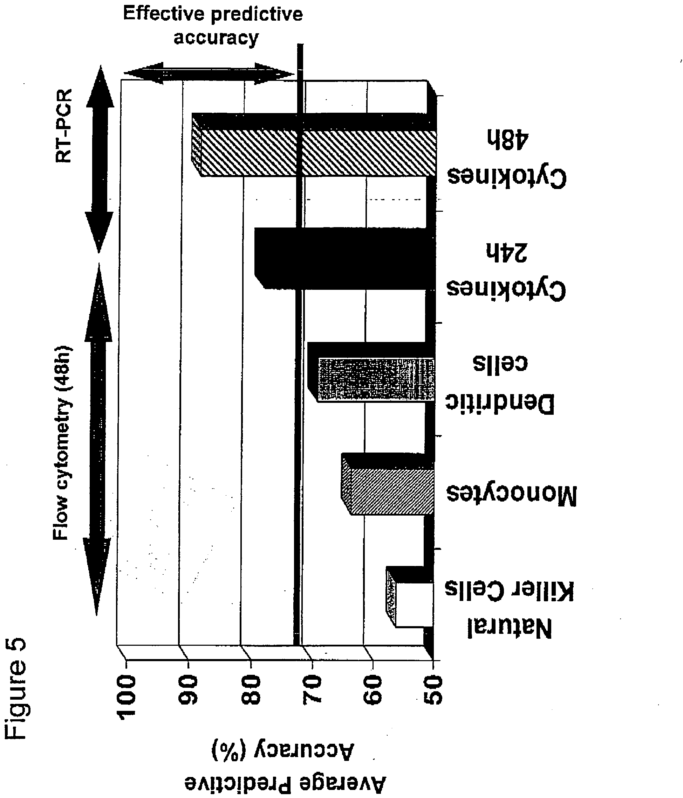

The most consistent results were obtained from the RT PCR results. Figure 4 shows the data obtained from three subjects, which demonstrates the somewhat heterogeneous patterns of change in the profiles. However, when subjected to the neural network analysis described above. the algorithm achieved a good level of identification of infected sample over uninfected controls (Figure 5).

## Claims

1. Method for screening a biological sample to detect early stages of infection, SIRS or sepsis comprising the steps of:
a) detecting expression of a set of informative biomarkers by RT-PCR and/or detecting expression of a set of informative cell surface biomarkers by means of flow cytometry and/or monitoring a set of standard clinical measurements
b) analysing the results of detection by means of neural network or multivariate statistical analysis
c) classifying said sample according to the likelihood and timing of the development of overt infection.

2. Method according to claim 1, wherein analysis of the results yields a prediction of a probability of clinical SIRS or sepsis developing.

3. Method according to claim 1, wherein analysis of the results yields a binary yes/no prediction of clinical SIRS or sepsis developing.

4. Method according to either of claims 2 or 3, wherein if the development of clinical SIRS or sepsis is predicted, the results are subjected to a second analysis to determine the likely timing and/or severity of the clinical disease.

5. Method according to any of claims 1 to 4 wherein the set of informative biomarkers the expression of which is detected by means of RT-PCR consists of at least 6 selected from the list consisting of FasL, MCP-1, TNFα, IL-1β, IL-6, IL-8, IL-10, INF-α and INF-γ

6. Method of claim 5 wherein the expression of at least 7 biomarkers is detected.

7. Method according to any of claims 1 to 4, wherein the set of informative cell surface biomarkers the expression of which is detected by means of flow cytometry consists of at least two selected from the list consisting of CD31, HLA-DR, CD54, CD11b, CD62L, CD 25, CD69 and CD80 and CD97

8. Method according any of claims 1 to 4, wherein the standard Clinical measurements analysed are selected from the list consisting of temperature, heart rate, total and differential white blood cell count (monocytes, lymphocytes, granulocytes, neutrophils), platelet count, serum creatinine, urea, lactate, base excess, pO₂, HCO₃⁻, and C-reactive protein.

9. Method of any of claims 1 to 8 wherein analysis is by means of multivariate statistical analysis comprising principle component analysis and/or discriminant function analysis.

10. Method of claim 9 wherein the multivariate statistical analysis comprises discriminant function analysis

11. Method of any of claims 1 to 8 wherein analysis is by means of neural network.

12. Method of claim 10 wherein the neural network is a multilayered perceptron neural network.

13. Method of any of claims 9 to 11, wherein the analysis is capable of correctly predicting clinical SIRS or sepsis in greater than 80% of cases.

14. System for screening a biological sample to detect early stages of infection, SIRS or sepsis comprising: a means of extracting and purifying RNA from cells in said sample, a thermal cycler or other means to amplify selected RNA sequences by means of reverse transcription polymerase chain reaction (RT-PCR), a means of detecting and quantifying the results of said RT-PCR, a computer-based neural network trained so as to be able to analyse such results and a display means whereby the conclusion of the neural network analysis may be communicated to an operator.

15. System for screening a biological sample to detect early stages of SIRS or sepsis comprising:
a means of labelling specific cell surface markers and quantifying expression of said markers, a means of detecting and quantifying the results of said labelling and quantification, a computer-based neural network trained so as to be able to analyse such results and a display means whereby the conclusion of the neural network analysis may be communicated to an operator.
Preferably, the labelling is by means of labelled antibodies or antibody fragments and the quantification is by means of fluorescence-activated cell sorting (FACS) or other form of flow cytometry.

16. System according to either of claims 13 or 14 comprising means of performing both RT-PCR and FACS analysis.

17. System according to any of claims 13 to 15 further comprising means to acquire and analyse data from standard clinical measurements.

18. Analysis according to the method of any of claims 1 to 12 or using the system of any of claims 13 to 16 for the preparation of a diagnostic means for the diagnosis of infection, SIRS or sepsis.

19. Use of a method according to any of claims 1 to 12 or the system according to any of claims 13 to 16 for the preparation of a diagnostic means for the diagnosis of infection, SIRS or sepsis.
